# EUROPÄISCHE PATENTSCHRIFT

(11) **EP 2 480 644 B1**
(45) Veröffentlichungstag und Bekanntmachung des Hinweises auf die Patenterteilung: **04.12.2013**
(21) Anmeldenummer: 10751579.3
(22) Anmeldetag: 03.09.2010
(51) Int. Cl.: C11C 3/04, C10L 1/02, C07C 67/03

(54) **KONTINUIERLICHES UMESTERUNGSVERFAHREN**
CONTINUOUS TRANSESTERIFICATION METHOD
PROCÉDÉ DE TRANSESTÉRIFICATION CONTINU

(30) Priorität: 22.09.2009 DE 102009042522
(43) Veröffentlichungstag der Anmeldung: 01.08.2012
(73) Patentinhaber: Clariant Finance (BVI) Limited, Road Town, Tortola (VG)
(72) Erfinder: KRULL, Matthias, 55296 Harxheim (DE); MORSCHHÄUSER, Roman, 55122 Mainz (DE)
(74) Vertreter: Mikulecky, Klaus
(86) Internationale Anmeldenummer: PCT/EP2010/005428
(87) Internationale Veröffentlichungsnummer: WO 2011/035853

(56) Entgegenhaltungen:
- EP-A1- 1 849 854
- GB-A- 2 361 918
- LERTSATHAPORNSUK V ET AL: "Microwave assisted in continuous biodiesel production from waste frying palm oil and its performance in a 100 kW diesel generator", FUEL PROCESSING TECHNOLOGY, ELSEVIER BV, NL, Bd. 89, Nr. 12, 1. Dezember 2008 (2008-12-01), Seiten 1330-1336, XP025681095, ISSN: 0378-3820, DOI: DOI:10.1016/J.FUPROC.2008.05.024 [gefunden am 2008-07-21]
- AZCAN N ET AL: "Microwave assisted transesterification of rapeseed oil", FUEL, IPC SCIENCE AND TECHNOLOGY PRESS, GUILDFORD, GB, Bd. 87, Nr. 10-11, 1. August 2008 (2008-08-01), Seiten 1781-1788, XP022611169, ISSN: 0016-2361, DOI: DOI:10.1016/J.FUEL.2007.12.004 [gefunden am 2008-01-03]
- AZCAN ET AL: "Alkali catalyzed transesterification of cottonseed oil by microwave irradiation", FUEL, IPC SCIENCE AND TECHNOLOGY PRESS, GUILDFORD, GB, Bd. 86, Nr. 17-18, 30. Oktober 2007 (2007-10-30), Seiten 2639-2644, XP022322088, ISSN: 0016-2361, DOI: DOI:10.1016/J.FUEL.2007.05.021

## Beschreibung

Die vorliegende Erfindung betrifft ein kontinuierliches Verfahren zur Herstellung von Fettsäureestern durch Umesterung von Fettsäureestern mehrwertiger Alkohole unter Mikrowellenbestrahlung im technischen Maßstab.

Ester organischer Säuren sind eine industriell bedeutsame Stoffgruppe, die vielfältige Verwendung zum Beispiel als chemischer Rohstoff beispielsweise für die Herstellung nichtionischer, biologisch abbaubarer Tenside oder auch als regenerativer Brennstoff (Biodiesel) findet. Eine gebräuchliche Methode zur Herstellung von Estern sind Umesterungsreaktionen, bei denen ein Ester durch Austausch des an eine Säuregruppe gebundenen Alkoholrestes in einen anderen Ester überführt wird.

Besondere Aufmerksamkeit haben in letzter Zeit Verfahren zur Herstellung von Fettsäuremethylestern aus Triglyceriden und ihre Verwendung als Biodiesel erlangt. Die Sorge um den Erhalt der Umwelt einerseits und das Bedürfnis nach einer langfristigen, sicheren und nachhattigen Energieversorgung andererseits haben den Bedarf an derartigen Energieträgern stark steigen lassen. Zudem fallen weltweit regelmäßig große Mengen an Abfallfetten zum Beispiel in (Fast Food-) Restaurants und der Lebensmittelindustrie an. Nach Umwandlung dieser Altfette in Biodiesel können diese als Brennstoff eingesetzt werden, wodurch die Entsorgungskosten für Altfette reduziert werden. Aber auch andere Öle und Fette können auf gleiche Weise in Biodiesel überführt werden.

Bei der Umwandlung von Ölen und Fetten in Biodiesel wird üblicherweise eine Umesterung vorgenommen, bei der das Glycerin der hauptsächlich aus Triglyceriden bestehenden Öle und Fette gegen einen einwertigen, bevorzugt niederen Alkohol wie beispielsweise Methanol oder Ethanol ausgetauscht wird. Die Umesterung ist eine Gleichgewichtsreaktion, die in der Regel bereits durch Mischen der Reaktanden ausgelöst wird. Die Reaktion verläuft jedoch so langsam, dass für kommerzielle Zwecke ein längeres Erwärmen und die Zugabe eines Katalysators zur Beschleunigung der Reaktion erforderlich sind. In neueren Publikationen werden zunehmend Verfahren offenbart, die unter erhöhten Temperaturen und/oder Drücken arbeiten. Das bei der Umesterung freigesetzte Glycerin wird durch Phasentrennung abgetrennt und überschüssiger Alkohol nach Abdestillieren rezykliert. Abschließend werden die erhaltenen Ester beispielsweise durch Waschen mit Wasser, Vakuumtrocknung und/oder Filtration aufgereinigt. Bei der üblicherweise angewandten alkalischen Katalyse mit Alkalialkoholaten ist auf möglichst weitgehende Wasserfreiheit zu achten, da Wasser zu Esterhydrolyse und damit verbundener Seifenbildung mit dem Katalysator führt. Auch dürfen die eingesetzten Triglyceride nur einen sehr geringen Gehalt an freien Fettsäuren aufweisen, da die ansonsten entstehenden Seifen in der Folge die Abtrennung von Glycerin wie auch die weiteren Reinigungsschritte erschweren.

WO 2009/002880 offenbart ein Verfahren zur Herstellung von Fettsäurealkylestern unter nahezu kritischen oder überkritischen Bedingungen in einem druckfesten Behälter. Das Erhitzen des Reaktionsgemischs erfolgt dabei über die Wandung des Behälters.

EP 1 884 559 A offenbart ein kontinuierliches Verfahren zur Umesterung von Triglyceriden in einem Reaktionsrohr bei 260 - 420 °C unter erhöhtem Druck, bevorzugt oberhalb 90 bar, so dass sich Methanol im überkritischen Zustand befindet, in Gegenwart eines immobilisierten Katalysators. Es werden Verweilzeiten im Reaktionsrohr von mindestens 10 Minuten benötigt, um annähernd quantitative Umsätze zu erreichen.

Für das Up-Scaling in einen industriell interessanten Maßstab gibt es für Verfahren wie das in der EP 1 884 559 beschriebene verschiedene Möglichkeiten. Zum einen kann die Strömungsgeschwindigkeit im Reaktionsrohr erhöht werden, was jedoch zur Erzielung schneller Heizraten hohe Manteltemperaturen erfordert. Zudem erfordert dabei die zur Erzielung hoher Umsätze benötigte Verweilzeit des Reaktionsguts bei Reaktionstemperatur üblicherweise auch eine Verlängerung des Reaktionsrohres. Zum anderen kann bei konstanter Strömungsgeschwindigkeit der Durchmesser des Reaktionsrohres vergrößert werden, was ebenfalls eine Erhöhung der Manteltemperatur zur Gewährleistung der notwendigen Reaktionstemperatur erfordert. Die erhöhten Temperaturen an den Rohrwandungen führen in beiden Fällen durch lokale Überhitzungen an diesen Heizflächen oftmals zu Zersetzungsreaktionen wie Decarboxylierung der Fettsäuren, Dehydratisierung der Polyole und/oder unkontrollierte Polymerisation insbesondere der ungesättigten Anteile der Triglyceride und somit zu verminderten Ausbeuten. Moderate Manteltemperaturen dagegen erfordern zur Erreichung der Zieltemperatur lange Verweilzeiten im Reaktionsrohr und damit niedrige Strömungsgeschwindigkeiten und/oder entsprechend lange Rohre. Während eines solchen langsamen Aufheizens werden bei vielen Reaktionen ebenfalls unerwünschte Nebenreaktionen beobachtet. In allen Fällen wird zudem das Reaktionsvolumen stark vergrößert, was erhöhte Sicherheitsvorkehrungen bei der Durchführung eines solchen Verfahrens erfordert.

Ein neuerer Ansatz zur Umesterung von Triglyceriden ist die durch Mikrowellen unterstützte Umsetzung von Triglyceriden mit niederen Alkoholen wie Methanol, mit der die Reaktion beschleunigt werden kann.

Mazzocchia et al. (C. R. Chemie, 2004, 7, 601-605) offenbaren mikrowellenunterstützte Umesterungen von Triglyceriden mit Methanol unter heterogener Katalyse durch Zeolithe. Dabei werden bei 170 °C und zweistündiger Bestrahlung im geschlossenen Gefäß jedoch nur moderate Umsätze erzielt.

Saifuddin et al. (Malaysian J. Chem. 2004, Vol. 6, 77-82) offenbaren ein Verfahren zur Herstellung von Fettsäureethylestern durch Umesterung von Triglyceriden mit Ethanol. Durch Mikrowellenbestrahlung wird dabei gegenüber rein thermischer Umsetzung eine deutliche Beschleunigung der Umesterung erzielt, ohne dass ein Einfluss auf die Gleichgewichtslage gefunden wurde. Die Reaktionstemperatur wurde auf 60 °C begrenzt, um Zersetzungen durch Überhitzung zu vermeiden.

Leadbeater et al. (Energy & Fuels, 2006, Vol. 20, 2281-2283) offenbaren Versuche zur Herstellung von Fettsäuremethylestem unter Mikrowellenbestrahlung und Katalyse durch KOH, wobei die diskontinuierliche Umesterung größer Volumen von bis zu 5 I in einem MultimodeMikrowellenapplikator bei Atmosphärendruck unter Rückfluss durchgeführt wird.

US 2005/0274065 offenbart Prozesse, bei denen Triglyceride mit Alkoholen in Gegenwart von Katalysatoren und/oder unter dem Einfluss von Mikrowellenenergie umgeestert werden. Dabei wird in einer speziellen Ausführungsform das sich in einer Vorlage befindliche Reaktionsgut kontinuierlich umgepumpt und dabei durch einen in einem Mikrowellen-Applikator befindlichen, gerührten Behälter geführt. Nach mehrmaligem Durchfahren des MikrowellenApplikators werden hohe Umesterungsgrade erzielt.

Das Scale-Up derartiger mikrowellenunterstützter Umesterungen aus dem Labor in einen technischen Maßstab und damit die Entwicklung von Anlagen, die für eine Produktion von mehreren Tonnen wie beispielsweise mehreren zehn, mehreren hundert oder mehreren tausend Tonnen pro Jahr mit für großtechnische Anwendungen interessanten Raum-Zeit-Ausbeuten geeignet sind, konnte bisher jedoch nicht realisiert werden. Ursache dafür ist zum einen die üblicherweise auf einige Millimeter bis wenige Zentimeter begrenzte Eindringtiefe von Mikrowellen in das Reaktionsgut, was insbesondere in Batch-Verfahren durchgeführte Reaktionen auf kleine Gefäße beschränkt oder in gerührten Reaktoren zu sehr langen Reaktionszeiten führt. Einer für die Bestrahlung großer Substanzmengen mit Mikrowellen wünschenswerten Erhöhung der Feldstärke sind insbesondere in den bisher bevorzugt zum Scale-Up chemischer Reaktionen eingesetzten Multimode-Geräten durch dann auftretende Entladungsvorgänge (Plasrnenbildung) enge Grenzen gesetzt. Weiterhin bereitet die in diesen Multimode-Mikrowellengeräten zu lokalen Überhitzungen des Reaktionsgutes führende, durch mehr oder weniger unkontrollierte Reflektionen der in den Mikrowellenofen eingestrahlten Mikrowellen an dessen Wänden und dem Reaktionsgut verursachte Inhomogenität des Mikrowellenfeldes Probleme bei der Maßstabsvergrößerung. Zudem bereitet dabei der sich während der Reaktion oftmals ändernde Mikrowellen-Absorptionskoeffizient des Reaktionsgemischs Schwierigkeiten hinsichtlich einer sicheren und reproduzierbaren Reaktionsführung.

Breccia et al. (J. Microwave Power Elecromag. Energy 1999, 34, 3-8) offenbaren die kontinuierliche Umesterung von Pflanzenölen in Gegenwart verschiedener Katalysatoren unter Mikrowellenbestrahlung. Dabei wird das Reaktionsgemisch durch eine in einen Multimode-Mikrowellenofen montierte Glaswendel geführt, wobei das Reaktionsgemisch bei einer Verweilzeit im Mikrowellenfeld von 2 Minuten die Siedetemperatur des Lösemittels erreicht.

WO 03/014272 offenbart ein Verfahren zur Herstellung von Fettsäuremethylestern aus Triglyceriden und Methanol unter Mikrowellenbestrahlung sowie eine Vorrichtung zur kontinuierlichen Durchführung des Verfahrens, bei dem die Umesterung in einem gerührten Stahlzylinder von etwa 120 cm Länge stattfindet, wobei die Mikrowellenstrahlung mittels einer Vielzahl an Magnetrons und Wellenleitern in das Reaktionsgefäß eingekoppelt wird.

WO 90/03840 offenbart ein kontinuierliches Verfahren zur Durchführung verschiedener chemischer Reaktionen wie beispielsweise Umesterungen in einem kontinuierlichen Labor-Mikrowellenreaktor. Die im Multimode betriebene Mikrowelle erlaubt jedoch kein Up-Scaling in den großtechnischen Bereich. Ihr Wirkungsgrad bezüglich der Mikrowellenabsorption des Reaktionsguts ist auf Grund der in Multimode-Mikrowellenapplikatoren auf den Applikatorraum mehr oder weniger homogen verteilten und nicht auf die Rohrschlange fokussierten Mikrowellenenergie niedrig. Eine starke Erhöhung der eingestrahlten Mikrowellenleistung kann zu unerwünschten Plasma-Entladungen oder zu so genannten thermischen Runaway-Effekten führen. Weiterhin machen die als Hot-Spots bezeichneten, sich zeitlich verändernden räumlichen Inhomogenitäten des Mikrowellenfeldes eine sichere und reproduzierbare Reaktionsführung im großen Maßstab unmöglich.

Leadbeater et al. (Energy & Fuels, 2007, 21, (3), pp 1777-1781) offenbaren die Umesterung von Triglyceriden mit Methanol in einem kontinuierlich betriebenen Rührgefäß, das in einem Multimode-Mikrowellenapplikator montiert ist, mit bis zu 4 l Volumen unter Atmosphärendruck und einer Flussrate von bis zu 7,2 l/min. Dabei wird von einem im Vergleich zu konventionell geheizten Umesterungen vergleichsweise geringen Energiebedarf berichtet. Die so hergestellten Methylester erhalten durch das zum Verhindern der Anreicherung von Glycerin im Reaktionsgefäß erforderliche Rühren und die damit verbundene Rückvermischung des Reaktionsgutes im Reaktionsgefäß jedoch noch vergleichsweise hohe Mengen an Di- und Triglyceriden, die die für Biodiesel in der DIN EN 14214 festgelegten Grenzwerte deutlich überschreiten. Sie sind dementsprechend nicht als Biodiesel vermarktbar. Zudem ist das Verfahren bedingt durch das große Reaktionsvolumen sicherheitstechnisch problematisch und zudem bedingt durch die begrenzte Eindringtiefe von Mikrowellen in das Reaktionsgut und den begrenzten Energieeintrag in Monomode-Mikrowellenöfen nicht beliebig zu vergrößern.

Lertsathapornsuk et al. (Fuel Processing Technology, 2008, 89, 1330-1336) offenbaren ein kontinuierliches Verfahren zur Herstellung von Estern, in dem ein Palmöl mit Ethanol unter Mikrowellenbestrahlung in einem Reaktionsrohr zu Biodiesel umgesetzt wird.

Die Bestrahlung des Reaktionsgemischs mit Mikrowellen erfolgt in einem weitgehend mikrowellentransparenten Reaktionsrohr innerhalb eines Hohlleiters.

Weiterhin sind Monomode- bzw. Singlemode-Mikrowellenapplikatoren bekannt, bei denen mit einem einzigen Wellen-Modus gearbeitet wird, der sich nur in einer Raumrichtung ausbreitet und durch exakt dimensionierte Wellenleiter auf das Reaktionsgefäß fokussiert wird. Diese Geräte erlauben zwar höhere lokale Feldstärken, sind bisher aber auf Grund der geometrischen Anforderungen (z. B. ist die Intensität des elektrischen Feldes an seinen Wellenbergen am größten und geht an den Knotenpunkten gegen Null) auf kleine Reaktionsvolumina (≤ 50 ml) im Labormaßstab beschränkt.

Es wurde daher ein Verfahren zur Umesterung von Estern gesucht, bei dem ein Carbonsäurepolyolester mit einem einwertigen Alkohol unter Mikrowellenbestrahlung auch im technischen Maßstab zu einem Ester aus Carbonsäure und dem einwertigen Alkohol umgesetzt werden kann. Dabei sollen bei möglichst kurzen Reaktionszeiten möglichst hohe, das heißt bis zu quantitative Umsetzungsraten erzielt werden. Das Verfahren soll weiterhin eine möglichst energiesparende Herstellung des Esters aus Carbonsäure und einwertigem Alkohol ermöglichen, das heißt, die eingesetzte Mikrowellenleistung soll möglichst quantitativ vom Reaktionsgut absorbiert werden und das Verfahren somit einen hohen energetischen Wirkungsgrad bieten. Dabei sollen - neben dem Polyol - keine bzw. nur untergeordnete Mengen an Nebenprodukten anfallen. Das Verfahren soll weiterhin die Verarbeitung von Ölen und Fetten mit einem erhöhten Anteil an freien Fettsäuren ermöglichen. Die hergestellten Ester aus Carbonsäure und einwertigem Alkohol sollen ferner eine geringe Eigenfärbung aufweisen. Zudem soll das Verfahren eine sichere und reproduzierbare Reaktionsführung gewährleisten.

Überraschenderweise wurde gefunden, dass die Umesterung von Polyolestern durch Umsetzung von Polyolestern mit Alkoholen in einem kontinuierlichen Verfahren durch nur kurzzeitiges Erhitzen mittels Bestrahlung mit Mikrowellen in einem Reaktionsrohr, dessen Längsachse sich in der Ausbreitungsrichtung der Mikrowellen eines Monomode-Mikrowellenapplikators befindet, in technisch relevanten Mengen durchgeführt werden kann. Dabei werden auch im eingesetzten Polyolester enthaltene Anteile freier Fettsäuren insbesondere bei saurer Katalyse in die entsprechenden Ester überführt. Die in den Mikrowellenapplikator eingestrahlte Mikrowellenenergie wird dabei praktisch quantitativ vom Reaktionsgut absorbiert. Das erfindungsgemäße Verfahren beisitzt zudem eine hohe Sicherheit bei der Durchführung und bietet eine hohe Reproduzierbarkeit der eingestellten Reaktionsbedingungen. Die nach dem erfindungsgemäßen Verfahren hergestellten Ester zeigen eine im Vergleich zu nach konventionellen Herstellverfahren ohne zusätzliche Verfahrensschritte nicht zugängliche hohe Reinheit und niedrige Eigenfärbung.

Gegenstand der Erfindung ist ein kontinuierliches Verfahren zur Herstellung von Estern, in dem mindestens ein Polyolester der Formel (I)

(R¹-COO)ₘR² (I)

worin
- R¹: für Wasserstoff oder einen gegebenenfalls substituierten Kohlenwasserstoffrest mit 1 bis 50 Kohlenstoffatomen,
- R²: für einen gegebenenfalls substituierten Kohlenwasserstoffrest mit 2 bis 10 Kohlenstoffatomen und
- m: für eine Zahl von 2 bis 10 steht und kleiner oder gleich der Zahl der Kohlenstoffatome in R² ist,
mit mindestens einem einwertigen Alkohol der Formel (II)

R³-OH (II)

worin
- R³: für einen gegebenenfalls substituierten Kohlenwasserstoffrest mit 1 bis 30 C-Atomen steht,
unter Mikrowellenbestrahlung in einem Reaktionsrohr, dessen Längsachse sich in der Ausbreitungsrichtung der Mikrowellen eines Monomode-Mikrowellenapplikators befindet, zu mindestens einem Ester der Formel (III)

R¹-COO-R³ (III)

worin
- R¹ und R³: die oben angegebenen Bedeutungen haben,
umgesetzt wird.

Erfindungsgemäß bevorzugte Ester der Formel (I) leiten sich von Carbonsäuren der Formel (IV)

R¹COOH (IV)

und Polyolen der Formel (V)

R²(OH)ₘ (V)

ab, wobei R¹, R² und m die oben angegebenen Bedeutungen haben, aus denen sie sich nach bekannten Methoden wie beispielsweise durch Kondensation herstellen lassen oder in biochemischen Prozessen anfallen.

Unter Carbonsäuren (IV) werden hier allgemein Verbindungen verstanden, die mindestens eine Carboxylgruppe an einem gegebenenfalls substituierten Kohlenwasserstoffrest mit 1 bis 50 C-Atomen besitzen sowie Ameisensäure. In einer bevorzugten Ausführungsform ist der Kohlenwasserstoffrest R³ ein aliphatischer Kohlenwasserstoffrest und insbesondere ein unsubstituierter Alkyl- oder Alkenylrest. In einer weiteren bevorzugten Ausführungsform trägt der aliphatische Kohlenwasserstoffrest einen oder mehrere wie beispielsweise zwei, drei, vier oder mehr weitere Substituenten. Geeignete Substituenten sind beispielsweise Halogenatome, C₁-C₅-Alkoxy- wie beispielsweise Methoxy-, Poly(C₁-C₅-Alkoxy)alkyl-, Keto-, Amid-, Cyano-, Nitril-, Nitro- und/oder Arylgruppen mit 5 bis 20 Kohlenstoffatomen wie beispielsweise Phenylgruppen, mit der Maßgabe, dass diese Substituenten unter den Reaktionsbedingungen stabil sind und keine Nebenreaktionen wie beispielsweise Eliminierungsreaktionen eingehen. Die C₅-C₂₀-Arylgruppen können ihrerseits wiederum Substituenten wie beispielsweise Halogenatome, C₁-C₂₀-Alkyl-, C₂-C₂₀-Alkenyl-, C₁-C₅-Alkoxy- wie beispielsweise Methoxy-, Amid-, Cyano-, Nitril-, und/oder Nitrogruppen tragen. Der Kohlenwasserstoffrest trägt jedoch höchstens so viele Substituenten wie er Valenzen hat.

Erfindungsgemäß besonders bevorzugt sind Carbonsäureester der Formel (I), die sich von aliphatischen Carbonsäuren (IV) mit einem gegebenenfalls substituierten aliphatischen Kohlenwasserstoffrest R¹ mit 2 bis 40 C-Atomen und insbesondere mit 6 bis 30 C-Atomen wie beispielsweise mit 8 bis 24 C-Atomen ableiten. Sie können synthetischen oder bevorzugt natürlichen Ursprungs sein. Der aliphatische Kohlenwasserstoffrest kann auch Heteroatome wie beispielsweise Sauerstoff, Stickstoff, Phosphor und/oder Schwefel enthalten, bevorzugt jedoch nicht mehr als ein Heteroatom pro 2 C-Atome und insbesondere nicht mehr als ein Heteroatom pro 3 C-Atome.

Die aliphatischen Kohlenwasserstoffreste R¹ können linear, verzweigt oder zyklisch sein. Bevorzugt sind sie linear. Sofern sie verzweigt sind, befindet sich die Verzweigung bevorzugt an dem zur Carbonylgruppe benachbarten C-Atom oder am Kettenende. Die Estergruppe kann an einem primären, sekundären oder tertiären C-Atom gebunden sein. Bevorzugt ist sie an einem primären C-Atom gebunden. Die Kohlenwasserstoffreste können gesättigt oder, sofern ihr Kohlenwasserstoffrest R¹ mindestens 2 Kohlenstoffatome umfasst, auch ungesättigt sein. Bevorzugte ungesättigte Kohlenwasserstoffreste besitzen bevorzugt eine oder mehrere C=C-Doppelbindungen und besonders bevorzugt eine, zwei oder drei C=C-Doppelbindungen. Weiterhin bevorzugt tragen sie keine zur Estergruppe konjugierte C=C-Doppelbindung. So hat sich das erfindungsgemäße Verfahren besonders zur Umesterung von Polyolestern bewährt, die eine oder mehrere mehrfach ungesättigte Carbonsäuren enthalten, da die Doppelbindungen der ungesättigten Carbonsäuren unter den Reaktionsbedingungen des erfindungsgemäßen Verfahrens nicht angegriffen werden. Bevorzugte zyklische aliphatische Kohlenwasserstoffreste besitzen mindestens einen Ring mit vier, fünf, sechs, sieben, acht oder mehr Ringatomen.

In einer besonders bevorzugten Ausführungsform leiten sich die Polyolester (I) von Fettsäuren ab. Dabei steht R¹ für einen gegebenenfalls substituierten aliphatischen Kohlenwasserstoffrest mit 6 bis 50 C-Atomen. Besonders bevorzugt leiten sie sich dabei von Fettsäuren ab, die einen aliphatischen Kohlenwasserstoffrest mit 7 bis 30 C-Atomen und insbesondere mit 8 bis 26 C-Atomen wie beispielsweise mit 10 bis 22 C-Atomen tragen. In einer bevorzugten Ausführungsform ist der Kohlenwasserstoffrest der Fettsäure ein unsubstituierter Alkyl- oder Alkenylrest. In einer weiteren bevorzugten Ausführungsform trägt der Kohlenwasserstoffrest der Fettsäure einen oder mehrere wie beispielsweise zwei, drei, vier oder mehr weitere Substituenten.

Zur Umesterung gemäß dem erfindungsgemäßen Verfahren geeignete Polyolester (I) sind Ester beispielsweise von Ameisensäure, Essigsäure, Propionsäure, Buttersäure, iso-Buttersäure, Pentansäure, iso-Pentansäure, Pivalinsäure, Crotonsäure, Phenylessigsäure, (Methoxyphenyl)essigsäure, (Dimethoxyphenyl)essigsäure, 2 Phenylpropionsäure, 3-Phenylpropionsäure, Hexansäure, Cyclohexansäure, Heptansäure, Octansäure, Nonansäure, Neononansäure, Decansäure, Neodecansäure, Undecansäure, Neoundecansäure, Dodecansäure, Tridecansäure, Tetradecansäure, 12-Methyltridecansäure, Pentadecansäure, 13-Methyltetradecansäure, 12 Methyltetradecansäure, Hexadecansäure, 14-Methylpentadecansäure, Heptadecansäure, 15-Methylhexadecansäure, 14-Methylhexadecansäure, Octadecansäure, Iso-Octadecansäure, Icosansäure, Docosansäure und Tetracosansäure, Myristolein-, Palmitolein-, Hexadecadien-, Delta-9-cis-Heptadecen-, Öl-, Petroselin-, Vaccen-, Linol-, Linolen-, Gadolein-, Gondo-, Icosadien-, Arachidon-, Cetolein-, Eruca-, Docosadien- und Tetracosensäure sowie deren Mischungen. Weiterhin geeignet sind Ester von Carbonsäureestermischungen (IV), die aus natürlichen Fetten und Ölen wie beispielsweise Baumwollsamen-, Cocos-, Erdnuss-, Färberdistel-, Mais-, Palm-, Palmkern-, Raps-, Oliven-, Senfsamen, Soja-, Sonnenblumenöl sowie Talg-, Knochen-, Fischöl und deren Mischungen gewinnbar sind.

In einer bevorzugten Ausführungsform leiten sich die Ester der Formel (I) von Polyolen der Formel (V) ab, bei denen der gegebenenfalls substituierte Kohlenwasserstoffrest R² für einen aliphatischen Rest steht. Dieser hat bevorzugt 2 bis 8, besonders bevorzugt 3 bis 6 und speziell 3 C-Atome. Der aliphatische Rest kann linear oder, sofern er mindestens 4 C-Atome umfasst, verzweigt oder zyklisch sein. Er kann weiterhin gesättigt oder, sofern er mindestens 3 C-Atome besitzt, ungesättigt sein. Bevorzugt ist der aliphatische Rest R² gesättigt. Gegebenenfalls kann der Kohlenwasserstoffrest R² Substituenten wie beispielsweise C₅-C₂₀-Arylgruppen tragen und/oder mit Heteroatomen wie beispielsweise Sauerstoff und/oder Stickstoff unterbrochen sein.

Weiterhin bevorzugt leiten sich die Ester der Formel (I) von Polyolen der Formel (V) ab, deren aliphatischer Rest R² zwei, drei, vier, fünf, sechs oder mehr Hydroxylgruppen trägt. Die Hydroxylgruppen können an benachbarten C-Atomen oder auch an weiter entfernten Kohlenstoffatomen des Kohlenwasserstoffrestes gebunden sein, jedoch höchstens eine OH-Gruppe pro Kohlenstoffatom. Die OH-Gruppen der den Estern (I) zu Grunde liegenden Polyole (V) können dabei vollständig oder auch nur teilweise verestert sein. Bevorzugt sind die OH-Gruppen vollständig oder zumindest weitgehend vollständig verestert. Weitgehend vollständig verestert bedeutet, das die Hydroxylzahl des eingesetzten Polyolesters (I) höchstens 50 mg KOH/g, bevorzugt 0,1 bis 30 mg KOH/g und insbesondere 1 bis 10 mg KOH/g wie beispielsweise 2 bis 5 mg KOH/g beträgt. Die Hydroxylgruppen der Polyole (V) können mit gleichen oder verschiedenen Carbonsäuren (IV) verestert sein.

Das erfindungsgemäße Verfahren eignet sich insbesondere zur Umsetzung von Polyolestern, die sich von Polyolen wie beispielsweise Ethylenglykol, 1,2-Propandiol, 1,3-Propandiol, Neopentylglykol, Diethylenglykol, Triethylenglykol, Polyethylenglykol, Glycerin, Sorbitan, Sorbitol, Pentaerythrit, Fructose und Glucose ableiten. In einer besonders bevorzugten Ausführungsform handelt es sich bei dem Polyol (V) um Glycerin.

Beispiele für erfindungsgemäß besonders geeignete Polyolester der Formel (I) sind Ester aus aliphatischen Carbonsäuren mit 6 bis 30 C-Atomen und Polyolen mit 3 bis 5 C-Atomen und insbesondere Triglyceride von Fettsäuren wie beispielsweise Triolein, Tristearin und biogene Öle und Fette. Des gleichen sind natürliche Fette und Öle wie beispielsweise Baumwollsamen-, Cocos-, Erdnuss-, Färberdistel-, Mais-, Jathropha-, Palmkem-, Raps-, Oliven-, Senfsamen, Soja-, Sonnenblumenöl sowie Talg-, Knochen- und Fischöl für die Umsetzung gemäß dem erfindungsgemäßen Verfahren besonderes geeignet.

In einer bevorzugten Ausführungsform handelt es sich bei dem Kohlenwasserstoffrest R³ um einen aliphatischen Rest. Bevorzugt hat dieser aliphatische Rest 1 bis 24, besonders bevorzugt 2 bis 18 und speziell 3 bis 6 C-Atome. Der aliphatische Rest kann linear, verzweigt oder zyklisch sein. Er kann weiterhin gesättigt oder, sofern er mindestens drei C-Atome hat, ungesättigt sein. Bevorzugt ist er gesättigt. Der Kohlenwasserstoffrest kann Substituenten wie beispielsweise Halogenatome, halogenierte Alkylreste, Methoxy-, C₁-C₅-Alkoxyalkyl-, Cyano-, Nitrils, Nitro- und/oder C₅-C₂₀-Arylgru ppen wie beispielsweise Phenylreste tragen. Die C₅-C₂₀-Arylreste können ihrerseits gegebenenfalls mit Halogenatomen, halogenierten Alkylresten, C₁-C₂₀-Alkyl-, C₂-C₂₀-Alkenyl-, C₁-C₅-Alkoxy- wie beispielsweise Methoxy-, Amid-, Cyano-, Nitril-, und/oder Nitrogruppen substituiert sein.

In einer weiteren bevorzugten Ausführungsform steht R³ für einen mit Heteroatomen unterbrochenen Alkylrest. Dabei enthält R³ jedoch höchstens ein Heteroatom pro zwei C-Atome. Bevorzugte Heteroatome sind Sauerstoff und Stickstoff. Besonders bevorzugtes Heteroatom ist Sauerstoff. Sofern der Rest R² Stickstoffatome enthält, so tragen diese Stickstoffatome keine aciden Protonen.

In einer weiteren bevorzugten Ausführungsform steht R³ für eine gegebenenfalls substituierte C₆-C₁₂-Arylgruppe oder eine gegebenenfalls substituierte heteroaromatische Gruppe mit 5 bis 12 Ringgliedern. Beispiele für geeignete Substituenten sind Halogenatome, halogenierte Alkylreste sowie Alkyl-, Alkenyl-, Alkoxy-, Amid-, Nitril- und Nitrogruppen.

Beispiele für bevorzugte aliphatische Reste R³ sind Methyl, Ethyl, n-Propyl, iso-Propyl, n-Butyt, iso-Butyl und tert.-Butyl, n-Hexyl, Cyclohexyl, n-Octyl, iso-Octyl, 2-Ethylhexyl, Decyl, Dodecyl, Tridecyl, Tetradecyl, Hexadacyl, Octadecyl und deren Mischungen. Beispiele für geeignete Alkohole der Formel II sind Methanol, Ethanol, 2-Methoxyethanol, n-Propanol, iso-Propanol, n-Butanol, iso-Butanol, tert.-Butanol, Pentanol, Neopentanol, n-Hexanol, iso-Hexanol, Cyclohexanol, Heptanol, n-Octanol, iso-Octanol, 2-Ethylhexanol, Decanol, Dodecanol, Tridecanol, iso-Tridecanol, Tetradecanol, Hexadecanol, Octadecanol und deren Mischungen.

In einer besonders bevorzugten Ausführungsform werden als Alkohole der Formel (II) unsubstituierte niedere aliphatische Alkohole mit 1 bis 6 C-Atomen und insbesondere mit 1 bis 3 C-Atomen wie beispielsweise Methanol, Ethanol und Propanol eingesetzt. Auch Mischungen verschiedener Alkohole (11) sind erfindungsgemäß geeignet. Insbesondere bevorzugt als Alkohol (II) sind Methanol und Ethanol.

Die Umsetzung der Polyolester der Formel (I) mit den Alkoholen (II) erfolgt bevorzugt mit einer mindestens equimolaren Menge des Alkohols (II) bezogen auf die Anzahl der Hydroxylgruppen im Polyol (V). Besonders bevorzugt erfolgt die Umsetzung mit molaren Verhältnissen von Alkohol (I) zu Hydroxylgruppen in Polyol (V) von 1,1 : 1 bis 50 : 1, insbesondere von 1,5 : 1 bis 15 : 1 und speziell von 2 : 1 bis 10 : 1 wie beispielsweise von 3 : 1 bis 8 : 1. Der überschüssige Alkohol wird nach erfolgter Umsetzung durch übliche Trennverfahren wie beispielsweise durch Destillation oder Flashen abgetrennt.

Das erfindungsgemäße Verfahren ist insbesondere geeignet zur Herstellung von Fettsäuremethylestern, Fettsäureethylestern, Fettsäurepropylestem und Fettsäurebutylestern wie beispielsweise Laurinsäuremethylester, Myristinsäuremethylester, Palmitinsäuremethylester, Margarinsäuremethylester, Stearinsäuremethylester, Ölsäuremethylester, Linolsäuremethylester, Linolensäuremethylester, Arachinsäuremethylester, Behensäuremethylester, Erucasäuremethylester, Laurinsäureethylester, Myristinsäureethylester, Palmitinsäureethylester, Margarir<säureethylester, Stearinsäureethylester, Ölsäureethylester, Linolsäureethylester, Linolensäureethylester, Arachinsäureethylester, Behensäureethylester, Erucasäureethylester, Laurinsäurepropylester, Myristinsäurepropylester, Palmitinsäurepropylester, Margarinsäurepropylester, Stearinsäurepropylester, Ölsäurepropylester, Linolsäurepropylester, Linolensäurepropylester, Arachinsäurepropylester, Behensäurepropylester, Erucasäurepropylester, Laurinsäurebutylester, Myristinsäurebutylester, Palmitinsäurebutylester, Margarinsäurebutylester, Stearinsäurebutylester, Ölsäurebutylester, Linolsäurebutylester, Linolensäurebutylester, Arachinsäurebutylester, Behensäurebutylester, Erucasäurebutylester und deren Mischungen wie beispielsweise Cocosfettsäuremethylester, Färberdistelfettsäuremethylester, Palmfettsäuremethylester, Rapsfettsäuremethylester, Olivenfettsäuremethylester, Sonnenblumenfettsäuremethylester, Sojafettsäuremethylester, Talgfettsäuremethylester, Jatrophafettsäuremethylester, Cocosfettsäureethylester, Färberdistelfettsäureethylester, Palmfettsäureethylester, Rapsfettsäureethylester, Olivenfettsäureethylester, Sonnenblumenfettsäureethylester, Sojafettsäureethylester, Talgfettsäureethylester, Jatrophafettsäureethylester, Cocosfettsäurepropylester, Färberdistelfettsäurepropylester, Palmfettsäurepropylester, Rapsfettsäurepropylester, Olivenfettsäurepropylester, Sonnenblumenfettsäurepropylester, Sojafettsäurepropylester, Talgfettsäurepropylester, Jatrophafettsäurepropylester, Cocosfettsäurebutylester, Färberdistelfettsäurebutylester, Palmfettsäurebutylester, Rapsfettsäurebutylester, Olivenfettsäurebutylester, Sonnenblumenfettsäurebutylester, Sojafettsäurebutylester, Talgfettsäurebutylester und Jatrophafettsäurebutylester.

In einer bevorzugten Ausführungsform wird zur Beschleunigung bzw. zur Vervollständigung der erfindungsgemäßen Umesterungsreaktionen in Gegenwart von Katalysatoren gearbeitet. Dabei können homogene Katalysatoren, heterogene Katalysatoren wie auch deren Mischungen eingesetzt werden.

Vorzugsweise arbeitet man dabei in Gegenwart eines basischen Katalysators oder Gemischen aus mehreren dieser Katalysatoren. Als basische Katalysatoren werden im Rahmen der vorliegenden Erfindung ganz allgemein solche basischen Verbindungen eingesetzt, die geeignet sind, die Umesterung von Carbonsäureestern mit Alkoholen zu beschleunigen. Beispiele geeigneter Katalysatoren sind anorganische und organische Basen wie beispielsweise Metallhydroxide, -oxide, -carbonate oder-alkoxide. In einer bevorzugten Ausführungsform wird der basische Katalysator ausgewählt aus der Gruppe der Hydroxide, Oxide, Carbonate oder Alkoxide von Alkali- oder Erdalkalimetallen. Dabei sind Lithiumhydroxid, Natriumhydroxid, Kaliumhydroxid, Natriummethoxid, Kaliummethoxid, Natriumcarbonat, Natrium-tert.-butanolat, Kalium-tert.-butanolat, Natriumoxid, Kaliumoxid und Kaliumcarbonat ganz besonders bevorzugt. Auch Cyanidionen sind als Katalysator geeignet. Diese Substanzen können in fester Form oder als Lösung wie beispielsweise als alkoholische Lösung eingesetzt werden. Besonders bevorzugte basische Katalysatoren sind Alkalialkoxide wie beispielsweise Natriummethoxid, Kaliummethoxid, Natriumethoxid, Kaliumethoxid, Natrium-tert.-butanolat und Kalium-tert.-butanolat. Besonders bevorzugt wird dabei das sich vom eingesetzten Alkohol (II) abgeleitete Alkalialkoxid eingesetzt.

Die Menge der eingesetzten basischen Katalysatoren hängt dabei von der Aktivität und Stabilität des Katalysators bei den gewählten Reaktionsbedingungen ab und ist der jeweiligen Reaktion anzupassen. Besonders bevorzugt werden katalytische Mengen der oben genannten, reaktionsbeschleunigend wirkenden Verbindungen eingesetzt, bevorzugt im Bereich zwischen 0,001 und 10 Gew.%, besonders bevorzugt im Bereich von 0,01 bis 5 Gew.-% wie beispielsweise zwischen 0,02 und 2 Gew.%, bezogen auf die eingesetzte Menge an Carbonsäureester (I) und Alkohol (II).

Weiterhin bevorzugt arbeitet man dabei in Gegenwart eines sauren anorganischen, metallorganischen oder organischen Katalysators oder Gemischen aus mehreren dieser Katalysatoren.

Als saure anorganische Katalysatoren im Sinne der vorliegenden Erfindung sind beispielsweise Schwefelsäure, Phosphorsäure, Phosphonsäure, hypophosphorige Säure, Aluminiumsulfathydrat, Alaun, saures Kieselgel und saures Aluminiumhydroxid zu nennen. Weiterhin sind beispielsweise Aluminiumverbindungen der allgemeinen Formel Al(OR¹⁵)₃ und Titanate der allgemeinen Formel Ti(OR¹⁵)₄ als saure anorganische Katalysatoren einsetzbar, wobei die Reste R¹⁵ jeweils gleich oder verschieden sein können und unabhängig voneinander gewählt sind aus C₁-C₁₀-Alkylresten, beispielsweise Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, sec.-Hexyl, n-Heptyl, n-Octyl, 2-Ethylhexy, n-Nonyl oder n-Decyl, C₃-C₁₂-Cycloalkylresten, beispielsweise Cyclopropyl, Cyclobutyl, Cyclopentyl, Cyclohexyl, Cycloheptyl, Cyclooctyl, Cyclononyl, Cyclodecyl, Cycloundecyl und Cyclododecyl; bevorzugt sind Cyclopentyl, Cyclohexyl und Cycloheptyl. Bevorzugt sind die Reste R¹⁵ in Al(OR¹⁵)₃ bzw. Ti(OR¹⁵)₄ jeweils gleich und gewählt aus Isopropyl, Butyl, Isobutyl und 2-Ethylhexyl.

Bevorzugte saure metallorganische Katalysatoren sind beispielsweise gewählt aus Dialkylzinnoxiden (R¹⁵)₂SnO, wobei R¹⁵ wie oben stehend definiert ist. Ein besonders bevorzugter Vertreter für saure metallorganische Katalysatoren ist Di-n-butylzinnoxid, das als sogenanntes Oxo-Zinn oder als Fascat^{®}-Marken kommerziell erhältlich ist.

Bevorzugte saure organische Katalysatoren sind saure organische Verbindungen mit beispielsweise Phosphatgruppen, Sulfonsäuregruppen, Sulfatgruppen oder Phosphonsäuregruppen. Besonders bevorzugte Sulfonsäuren enthalten mindestens eine Sulfonsäuregruppe und mindestens einen gesättigten oder ungesättigten, linearen, verzweigten und/oder zyklischen Kohlenwasserstoffrest mit 1 bis 40 C-Atomen und bevorzugt mit 3 bis 24 C-Atomen. Insbesondere bevorzugt sind aliphatische Sulfonsäuren und aromatische Sulfonsäuren, speziell alkylaromatische Mono-Sulfonsäuren mit einem oder mehreren C₁-C₂₈-Alkylresten und insbesondere solche mit C₃-C₂₂-Alkylresten. Geeignete Beispiele sind Methansulfonsäure, Butansulfonsäure, Benzolsulfonsäure, p-Toluolsulfonsäure, Xylolsulfonsäure, 2-Mesitylensulfonsäure, 4-Ethylbenzolsulfonsäure, Isopropylbenzolsulfonsäure, 4-Butylbenzolsulfonsäure, 4-Octylbenzolsulfonsäure; Dodecylbenzolsulfonsäure, Didodecylbenzolsulfonsäure, Naphthalinsulfonsäure. Auch saure Ionenaustauscher können als saure organische Katalysatoren eingesetzt werden, beispielsweise Sulfonsäuregruppen tragende Poly(styrol)-Harze, die mit etwa 2 mol-% Divinylbenzol vernetzt sind.

Besonders bevorzugte saure Katalysatoren für die Durchführung des erfindungsgemäßen Verfahrens sind Borsäure, Phosphorsäure, Polyphosphorsäure, Schwefelsäure, Methansulfonsäure, Alkylbenzolsulfonsäuren wie beispielswesie Dodecylbenzolsulfonsäure und Polystyrolsulfonsäuren. Insbesondere bevorzugt sind Titanate der allgemeinen Formel Ti(OR¹⁵)₄ und speziell Titantetrabutylat und Titantetraisopropylat.

Wünscht man saure anorganische, metallorganische oder organische Katalysatoren einzusetzen, so setzt man erfindungsgemäß bevorzugt 0,001 bis 10 Gew.-%, besonders bevorzugt 0,01 bis 5 Gew.-% wie beispielsweise zwischen 0,02 bis 2 Gew.-% Katalysator bezogen auf die eingesetzte Menge an Carbonsäureester und Alkohol ein.

In einer weiteren bevorzugten Ausführungsform wird die Mikrowellenbestrahlung in Gegenwart von festen Katalysatoren durchgeführt. Dabei wird der feste Katalysator in einem gegebenenfalls mit Lösemittel versetzten Edukt oder dem Reaktionsgemisch suspendiert oder vorteilhafterweise das gegebenenfalls mit Lösemittel versetzte Reaktionsgemisch über einen Festbettkatalysator geleitet und Mikrowellenstrahlung ausgesetzt. Geeignete feste Katalysatoren sind beispielsweise Zeolithe, Kieselgel, Montmorillonit und (teil)vernetzte Polystyrolsulfonsäure, die gegebenenfalls mit katalytisch aktiven Metallsalzen imprägniert sein können.

Wird die Umesterung unter Temperatur- und Druckbedingungen durchgeführt, unter denen sich der eingesetzte Alkohol (II) im überkritischen Zustand befindet, erfolgt die Umesterung in einer erfindungsgemäß bevorzugten Ausführungsform ohne Zugabe eines Katalysators.

Die erfindungsgemäße Umesterung erfolgt durch Vermischen von Carbonsäureester (I) und Alkohol (II) sowie gegebenenfalls Katalysator und nachfolgende Bestrahlung des Reaktionsgemischs mit Mikrowellen in einem Reaktionsrohr, dessen Längsachse sich in der Ausbreitungsrichtung der Mikrowellen in einem Monomode-Mikrowellenapplikators befindet.

Bevorzugt erfolgt die Bestrahlung des Reaktionsgemischs mit Mikrowellen in einem weitgehend mikrowellentransparenten Reaktionsrohr, das sich innerhalb eines mit einem Mikrowellengenerator verbundenen Hohlleiters befindet. Bevorzugt fluchtet das Reaktionsrohr axial mit der zentralen Symmetrieachse des Hohlleiters.

Der als Mikrowellenapplikator fungierende Hohlleiter ist bevorzugt als Hohlraumresonator ausgeformt. Bevorzugt wird die Länge des Hohlraumresonators so dimensioniert, dass sich in ihm eine stehende Welle ausbildet. Weiterhin bevorzugt werden die im Hohlleiter nicht absorbierten Mikrowellen an seinem Ende reflektiert. Durch Ausformung des Mikrowellenapplikators als Resonator vom Reflexionstyp werden eine lokale Erhöhung der elektrischen Feldstärke bei gleicher vom Generator zugeführter Leistung und eine erhöhte Energieausnutzung erzielt.

Der Hohlraumresonator wird bevorzugt im E₀₁ₙ-Mode betrieben, wobei n für eine ganze Zahl steht und die Anzahl der Feldmaxima der Mikrowelle entlang der zentralen Symmetrieachse des Resonators angibt. Bei diesem Betrieb ist das elektrische Feld in Richtung der zentralen Symmetrieachse des Hohlraumresonators gerichtet. Es hat im Bereich der zentralen Symmetrieachse ein Maximum und nimmt zur Mantelfläche hin auf den Wert null ab. Diese Feldkonfiguration liegt rotationssymmetrisch um die zentrale Symmetrieachse vor. Durch Verwendung eines Hohlraumresonators mit einer Länge, bei der n eine ganze Zahl ist, wird die Ausbildung einer stehenden Welle ermöglicht. Je nach der gewünschten Strömungsgeschwindigkeit des Reaktionsguts durch das Reaktionsrohr, der benötigten Temperatur und der benötigten Verweilzeit im Resonator wird die Länge des Resonators relativ zu der Wellenlänge der eingesetzten Mikrowellenstrahlung ausgewählt. Bevorzugt ist n eine ganze Zahl von 1 bis 200, besonders bevorzugt von 2 bis 100, insbesondere von 3 bis 50 von speziell 4 bis 20 wie beispielsweise drei, vier, fünf, sechs, sieben, acht, neun oder zehn.

Die E₀₁ₙ-Mode des Hohlraumresonators wird in Englischer Sprache auch als TM₀₁ₙ-Mode bezeichnet, siehe beispielsweise K. Lange, K.H. Löcherer, Taschenbuch der Hochfrequenztechnik", Band 2, Seite K21 ff.

Die Einstrahlung der Mikrowellenenergie in den als Mikrowellenapplikator fungierenden Hohlleiter kann über geeignet dimensionierte Löcher oder Schlitze erfolgen. In einer erfindungsgemäß besonders bevorzugten Ausführungsform erfolgt die Bestrahlung des Reaktionsgemischs mit Mikrowellen in einem Reaktionsrohr, das sich in einem Hohlleiter mit koaxialem Übergang der Mikrowellen befindet. Für dieses Verfahren besonders bevorzugte Mikrowelleneinrichtungen sind aus einem Hohlraumresonator, einer Koppeleinrichtung zum Einkoppeln eines Mikrowellenfeldes in den Hohlraumresonator und mit je einer Öffnung an zwei gegenüber liegenden Stirnwänden zum Hindurchführen des Reaktionsrohres durch den Resonator aufgebaut. Die Einkopplung der Mikrowellen in den Hohlraumresonator erfolgt bevorzugt über einen Koppelstift, der in den Hohlraumresonator hineinragt. Bevorzugt ist der Koppelstift als ein als Kopplungsantenne fungierendes, bevorzugt metallisches Innenleiterrohr ausgeformt. In einer besonders bevorzugten Ausführungsform ragt dieser Koppetstift durch eine der stirnseitigen Öffnungen in den Hohlraumresonator hinein. Besonders bevorzugt schließt sich das Reaktionsrohr an das Innenleiterrohr des koaxialen Übergangs an und speziell wird es durch dessen Hohlraum hindurch in den Hohlraumresonator geführt. Bevorzugt fluchtet das Reaktionsrohr axial mit einer zentralen Symmetrieachse des Hohlraumresonators. Dazu weist der Hohlraumresonator bevorzugt je eine zentrische Öffnung an zwei gegenüber liegenden Stirnwänden zum Hindurchführen des Reaktionsrohres auf.

Die Einspeisung der Mikrowellen in den Koppelstift bzw. in das als Kopplungsantenne fungierende Innenleiterrohr kann beispielsweise mittels einer koaxialen Anschlussleitung erfolgen. In einer bevorzugten Ausführungsform wird das Mikrowellenfeld über einen Hohlleiter dem Resonator zugeführt, wobei das aus dem Hohlraumresonator herausragende Ende des Koppelstifts in eine Öffnung, die sich in der Wand des Hohlleiters befindet, in den Hohlleiter hineingeführt ist und von dem Hohlleiter Mikrowellenenergie entnimmt und in den Resonator koppelt.

In einer speziellen Ausführungsform erfolgt die Bestrahlung des Reaktionsgemischs mit Mikrowellen in einem mikrowellentransparenten Reaktionsrohr, das sich axialsymmetrisch in einem E₀₁ₙ-Rundhohlleiter mit koaxialem Übergang der Mikrowellen befindet. Dabei wird das Reaktionsrohr durch den Hohlraum eines als Kopplungsantenne fungierenden Innenleiterrohres in den Hohlraumresonator geführt. In einer weiteren bevorzugten Ausführungsform erfolgt die Bestrahlung des Reaktionsgemischs mit Mikrowellen in einem mikrowellentransparenten Reaktionsrohr, das durch einen E₀₁ₙ-Hohlraumresonator mit axialer Einspeisung der Mikrowellen geführt wird, wobei die Länge des Hohlraumresonators so bemessen ist, dass sich n = 2 oder mehr Feldmaxima der Mikrowelle ausbilden. In einer weiteren bevorzugten Ausführungsform erfolgt die Bestrahlung des Reaktionsgemisches mit Mikrowellen in einem mikrowellentransparenten Reaktionsrohr, das durch einen E₀₁ₙ-Hohlraumresonator mit axialer Einspeisung der Mikrowellen geführt wird, wobei die Länge des Hohlraumresonators so bemessen ist, dass sich eine stehende Welle mit n = 2 oder mehr Feldmaxima der Mikrowelle ausbildet. In einer weiteren bevorzugten Ausführungsform erfolgt die Bestrahlung des Reaktionsgemischs mit Mikrowellen in einem mikrowellentransparenten Reaktionsrohr, das sich axialsymmetrisch in einem kreiszylindrischen E₀₁ₙ-Hohlraumresonator mit koaxialem Übergang der Mikrowellen befindet, wobei die Länge des Hohlraumresonators so bemessen ist, dass sich n = 2 oder mehr Feldmaxima der Mikrowelle ausbilden. In einer weiteren bevorzugten Ausführungsform erfolgt die Bestrahlung des Reaktionsgemisches mit Mikrowellen in einem mikrowellentransparenten Reaktionsrohr, das sich axialsymmetrisch in einem kreiszylindrischen E₀₁ₙ-Hohlraumresonator mit koaxialem Übergang der Mikrowellen befindet, wobei die Länge des Hohlraumresonators so bemessen ist, dass sich eine stehende Welle mit n = 2 oder mehr Feldmaxima der Mikrowelle ausbildet.

Mikrowellengeneratoren, wie beispielsweise das Magnetron, das Klystron und das Gyrotron sind dem Fachmann bekannt.

Die zur Durchführung des erfindungsgemäßen Verfahrens eingesetzten Reaktionsrohre sind bevorzugt aus weitgehend mikrowellentransparentem, hoch schmelzendem Material gefertigt. Besonders bevorzugt werden nichtmetallische Reaktionsrohre eingesetzt. Unter weitgehend mikrowellentransparent werden hier Werkstoffe verstanden, die möglichst wenig Mikrowellenenergie absorbieren und in Wärme umwandeln. Als Maß für die Fähigkeit eines Stoffes, Mikrowellenenergie zu absorbieren und in Wärme zu überführen wird oftmals der dielektrische Verlustfaktor tan δ = ε"/ε' herangezogen. Der dielektrische Verlustfaktor tan δ ist definiert als das Verhältnis aus dielektrischem Verlust ε" und Dielektrizitätskonstante ε'. Beispiele für tan δ-Werte verschiedener Materialien sind beispielsweise in D. Bogdal, Microwave-assisted Organic Synthesis, Elsevier 2005 wiedergegeben. Für erfindungsgemäß geeignete Reaktionsrohre werden Materialen mit bei 2,45 GHz und 25 °C gemessenen tan δ-Werten von unter 0,01, insbesondere unter 0,005 und speziell unter 0,001 bevorzugt. Als bevorzugte mikrowellentransparente und temperaturstabile Materialien kommen in erster Linie Werkstoffe auf mineralischer Basis wie beispielsweise Quarz, Aluminiumoxid, Saphir, Zirkonoxid, Siliziumnitrid und ähnliches in Betracht. Auch temperaturstabile Kunststoffe wie insbesondere Fluorpolymere wie beispielsweise Teflon, und technische Kunststoffe wie Polypropylen, oder Polyaryletherketone wie beispielsweise glasfaserverstärktes Polyetheretherketon (PEEK) sind als Rohrmaterialien geeignet. Um den Temperaturbedingungen während der Reaktion zu widerstehen haben sich insbesondere mit diesen Kunststoffen beschichtete Mineralien wie Quarz oder Aluminiumoxid als Reaktormaterialien bewährt.

Für das erfindungsgemäße Verfahren besonders geeignete Reaktionsrohre haben einen Innendurchmesser von einem Millimeter bis ca. 50 cm, insbesondere zwischen 2 mm und 35 cm und speziell zwischen 5 mm und 15 cm wie beispielsweise zwischen 10 mm und 7 cm. Unter Reaktionsrohren werden hier Gefäße verstanden, deren Verhältnis von Länge zu Durchmesser größer als 5, bevorzugt zwischen 10 und 100.000, besonders bevorzugt zwischen 20 und 10.000 wie beispielsweise zwischen 30 und 1.000 ist. Unter der Länge des Reaktionsrohres wird hier die Strecke des Reaktionsrohres verstanden, auf der die Mikrowellenbestrahlung erfolgt. In das Reaktionsrohr können Stromstörer und/oder andere Mischelemente eingebaut sein.

Für das erfindungsgemäße Verfahren besonders geeignete E₀₁-Hohlraumresonatoren haben bevorzugt einen Durchmesser, der mindestens der halben Wellenlänge der verwendeten Mikrowellenstrahlung entspricht. Bevorzugt beträgt der Durchmesser des Hohlraumresonators das 1,0- bis 10-fache, besonders bevorzugt das 1,1- bis 5-fache und insbesondere das 2,1- bis 2,6-fache der halben Wellenlänge der verwendeten Mikrowellenstrahlung. Bevorzugt hat der E₀₁-Hohlraumresonator einen runden Querschnitt, was auch als E₀₁-Rundhohlleiter bezeichnet wird. Besonders bevorzugt hat er eine zylindrische Form und speziell eine kreiszylindrische Form.

Das Reaktionsrohr ist üblicherweise am Einlass mit einer Dosierpumpe sowie einem Manometer und am Auslass mit einer Druckhaltevorrichtung und einer Kühlvorrichtung wie beispielsweise einem Wärmetauscher versehen. Damit sind Reaktionen in einem sehr weiten Druck- und Temperaturbereich möglich. In einer bevorzugten Ausführungsform wird das Reaktionsgut direkt nach Verlassen des Reaktionsrohres möglichst schnell auf Temperaturen unterhalb 120 °C, bevorzugt unterhalb 100 °C und speziell unterhalb 60 °C abgekühlt. Dies kann beispielsweise mittels Wärmetauscher oder adiabatischer Expansion erfolgen. Üblicherweise erfolgt die Entspannung des Reaktionsgemischs auf Atmosphärendruck, sie kann aber für anschließende Verfahrensschritte bzw. bei Verwendung spezieller Apparaturen auch auf höhere oder niedrigere Drücke erfolgen. So hat es sich beispielsweise bewährt, das Reaktionsgemisch zur Abtrennung von Lösemittel und/oder unumgesetzten Edukten auf Drücke deutlich unterhalb des Atmosphärendrucks zu entspannen. Die Abkühlung kann in Abhängigkeit von den Eigenschaften der umgesetzten Produkte und der vorgesehenen weiteren Verfahrensschritte vor oder auch nach Druckabsenkung oder bei einem dazwischen liegenden Druck erfolgen.

In einer besonders bevorzugten Ausführungsform wird das Reaktionsgemisch nach Passieren des Reaktionsrohres direkt, das heißt ohne Zwischenkühlung in eine isotherme Reaktionsstrecke überführt, in der es für eine gewisse Zeit auf Reaktionstemperatur gehalten wird. Erst nach Verlassen der Reaktionsstrecke wird das Reaktionsgemisch gegebenenfalls entspannt und abgekühlt. Unter der direkten Überführung aus dem Reaktionsrohr in die isothermen Reaktionsstrecke ist zu verstehen, dass zwischen Reaktionsrohr und Reaktionsstrecke keine aktiven Maßnahmen zum Zuführen und insbesondere zum Abführen von Wärme getroffen werden. Bevorzugt ist die Temperaturdifferenz zwischen Verlassen des Reaktionsrohres bis zum Eintritt in die Reaktionsstrecke kleiner als t 30 °C, bevorzugt kleiner ± 20 °C, besonders bevorzugt kleiner ± 10 °C und insbesondere kleiner ± 5 °C. In einer speziellen Ausführungsform entspricht die Temperatur des Reaktionsguts beim Eintritt in die Reaktionsstrecke der Temperatur beim Verlassen des Reaktionsrohres. Diese Ausführungsvariante ermöglicht eine schnelle und gezielte Erhitzung des Reaktionsgutes auf die gewünschte Reaktionstemperatur ohne partielle Überhitzung und sodann ein Verweilen bei dieser Reaktionstemperatur für einen definierten Zeitraum bevor es abgekühlt wird. So können eine erhöhte Raum-Zeit-Ausbeute, eine erhöhte energetische Effizienz und darüber hinaus ein sicheres und reproduzierbares Arbeiten erzielt werden.

Als isotherme Reaktionsstrecke kommen alle chemisch inerten Gefäße in Betracht, die ein Verweilen der Reaktionsgemische bei der im Reaktionsrohr eingestellten Temperatur ermöglichen. Unter isothermer Reaktionsstrecke wird verstanden, dass die Temperatur des Reaktionsgutes in der Reaktionsstrecke gegenüber der Eintrittstemperatur auf ± 30 °C, bevorzugt auf ± 20 °C, besonders bevorzugt auf ± 10 °C und insbesondere auf ± 5 °C konstant gehalten wird. Somit hat das Reaktionsgut beim Verlassen der Reaktionsstrecke eine Temperatur, die maximal ± 30 °C, bevorzugt ± 20 °C, besonders bevorzugt ± 10 °C und insbesondere ± 5 °C von der Temperatur beim Eintritt in die Reaktionsstrecke abweicht.

Neben kontinuierlich betriebenen Rührbehältern und Behälterkaskaden sind insbesondere Rohre als isotherme Reaktionsstrecke geeignet. Diese Reaktionsstrecken können aus verschiedenen Materialien wie beispielsweise Metallen, Keramik, Glas, Quarz oder Kunststoffen bestehen mit der Maßgabe, dass diese unter den gewählten Temperatur- und Druckbedingungen mechanisch stabil und chemisch inert sind. Besonders bewährt haben sich dabei thermisch isolierte Gefäße. Die Verweilzeit des Reaktionsguts in der Reaktionsstrecke kann beispielsweise über das Volumen der Reaktionsstrecke eingestellt werden. Bei Verwendung von Rührbehältern und Behälterkaskaden hat es sich gleichermaßen bewährt, die Verweilzeit über den Füllgrad der Behälter einzustellen.

In einer bevorzugten Ausführungsform wird als Reaktionsstrecke ein Rohr verwendet. Dabei kann es sich um eine Verlängerung des mikrowellentransparenten Heizrohres im Anschluss an die Heizzone oder auch ein separates, mit dem Heizrohr in Verbindung stehendes Rohr aus gleichem oder unterschiedlichem Material handeln. Über die Länge des Rohres und/oder seinen Querschnitt lässt sich bei gegebener Flussrate die Verweilzeit des Reaktionsgutes bestimmen. Das als Reaktionsstrecke fungierende Rohr ist im einfachsten Fall thermisch isoliert, so dass die beim Eintritt des Reaktionsgutes in die Reaktionsstrecke herrschende Temperatur in den oben gegebenen Grenzen gehalten wird. Dem Reaktionsgut kann in der Reaktionsstrecke aber auch beispielsweise mittels eines Wärmeträgers bzw. Kühlmediums gezielt Energie zu- oder abgeführt werden. Diese Ausführungsform hat sich insbesondere zum Anfahren des Verfahrens bewährt. So kann die Reaktionsstrecke beispielsweise als Rohrschlange oder Rohrbündel ausgestaltet sein, die sich in einem Heiz- oder Kühlbad befindet oder in Form eines Doppelmantelrohres mit einem Heiz- oder Kühlmedium beaufschlagt werden. Die Reaktionsstrecke kann sich auch in einem weiteren Mikrowellenapplikator befinden, in dem das Reaktionsgut nochmals mit Mikrowellen behandelt wird. Dabei können sowohl im Monomode- wie auch Multimode arbeitende Applikatoren zum Einsatz kommen.

Die Verweilzeit des Reaktionsgutes in der isothermen Reaktionsstrecke liegt üblicherweise zwischen 1 Sekunde und 10 Stunden, bevorzugt zwischen 10 Sekunden und 2 Stunden, besonders bevorzugt zwischen 20 Sekunden und 60 Minuten wie beispielsweise zwischen 30 Sekunden und 30 Minuten. Nach Verlassen der isothermen Reaktionsstrecke wird das Reaktionsgemisch dann wiederum möglichst schnell auf Temperaturen unterhalb 120 °C, bevorzugt unterhalb 100 °C und speziell unterhalb 60 °C abgekühlt, wobei wiederum die oben aufgeführten Vorrichtungen und Maßnahmen bevorzugt sind.

Die Herstellung des Reaktionsgemischs aus Ester (I), Alkohol (II) und gegebenenfalls Katalysator und/oder Lösemittel kann kontinuierlich, diskontinuierlich oder auch in semi-Batch-Prozessen durchgeführt werden. So kann das Reaktionsgemisch in einem vorgelagerten (semi)-Batch Prozess hergestellt werden wie beispielsweise in einem Rührbehälter. In einer bevorzugten Ausführungsform werden die Edukte Polyolester (I) und Alkohol (II), beide unabhängig voneinander gegebenenfalls mit Lösemittel verdünnt, erst kurz vor dem Eintritt in das Reaktionsrohr vermischt. So hat es sich insbesondere bei Einsatz nicht unbegrenzt miteinander mischbarer Edukte bewährt, die Mischung von Polyolester (I) und Alkohol (II) in einer Mischstrecke vorzunehmen, aus der das Reaktionsgemisch in das Reaktionsrohr gefördert wird. Weiterhin bevorzugt werden die Edukte dem erfindungsgemäßen Verfahren in flüssiger Form zugeführt. Dazu können höher schmelzende und/oder höher viskose Edukte beispielsweise in geschmolzenem Zustand und/oder mit Lösemittel versetzt beispielsweise als Lösung, Dispersion oder Emulsion eingesetzt werden. Ein Katalysator kann, sofern eingesetzt, einem der Edukte oder auch der Eduktmischung vor dem Eintritt in das Reaktionsrohr zugesetzt werden. Bevorzugt werden Katalysatoren in flüssiger Form, zum Beispiel als Lösung in einem der Edukte oder in einem unter den Reaktionsbedingungen inerten Lösemittel eingesetzt. Auch heterogene Systeme können nach dem erfindungsgemäßen Verfahren umgesetzt werden, wobei entsprechende technische Vorrichtungen zum Fördern des Reaktionsgutes erforderlich sind.

Das Reaktionsgemisch kann in das Reaktionsrohr entweder an dem durch das Innenleiterrohr geführte Ende eingespeist werden als auch an dem entgegen gesetzten Ende.

Durch Variation von Rohrquerschnitt, Länge der Bestrahlungszone (hierunter wird die Länge des Reaktionsrohres verstanden, in dem das Reaktionsgut Mikrowellenstrahlung ausgesetzt ist), Fließgeschwindigkeit, Geometrie des Hohlraumresonators sowie der eingestrahlten Mikrowellenleistung werden die Reaktionsbedingungen bevorzugt so eingestellt, dass die maximale Reaktionstemperatur schnellstmöglich erreicht wird und die Verweilzeit bei Maximaltemperatur so kurz bleibt, dass so wenig Neben- oder Folgereaktionen wie möglich auftreten. Das Reaktionsgut kann zur Vervollständigung der Reaktion, gegebenenfalls nach Zwischenkühlung und/oder Abtrennung von Komponenten wie beispielsweise Polyol und/oder Ergänzung von Reaktanden wie beispielsweise Alkohol und/oder Katalysator, mehrfach das Reaktionsrohr durchlaufen. Dabei können mit gleichem Ergebnis auch Kaskaden aus zwei, drei oder mehreren der Mikrowellenapplikatoren zum Einsatz kommen. Vielfach hat sich bewährt, wenn das Reaktionsprodukt unmittelbar nach Verlassen des Reaktionsrohres z. B. durch Mantelkühlung oder Entspannung abgekühlt wird.

Bevorzugt wird der durch die Mikrowellenbestrahlung bedingte Temperaturanstieg beispielsweise durch Regelung der Mikrowellenintensität, der Durchflussgeschwindigkeit und/oder durch Kühlung des Reaktionsrohres, beispielsweise durch einen Stickstoffstrom, auf maximal 500 °C begrenzt. Die Temperatur kann beispielsweise an der Oberfläche des Reaktionsrohres gemessen werden; bevorzugt wird sie am Reaktionsgut direkt nach dem Verlassen der Heizzone bestimmt. Besonders bewährt hat sich die Durchführung der Umsetzung bei Temperaturen zwischen 80 und maximal 400 °C, insbesondere zwischen 100 und 180 °C und speziell zwischen 120 und maximal 170 °C wie beispielsweise bei Temperaturen zwischen 130 und 160 °C.

Die Dauer der Mikrowellenbestrahlung hängt von verschiedenen Faktoren wie beispielsweise der Geometrie des Reaktionsrohres, der eingestrahlten Mikrowellenenergie, der eingesetzten Reaktanden und dem gewünschten Umsetzungsgrad ab. Üblicherweise wird die Mikrowellenbestrahlung über einen Zeitraum von weniger als 30 Minuten, bevorzugt zwischen 0,01 Sekunden und 15 Minuten, besonders bevorzugt zwischen 0,1 Sekunden und 10 Minuten und insbesondere zwischen einer Sekunde und 5 Minuten wie beispielsweise zwischen 5 Sekunden und 2 Minuten vorgenommen. Die Intensität (Leistung) der Mikrowellenstrahlung wird dabei so eingestellt, dass das Reaktionsgut beim Verlassen des Reaktionsrohres die gewünschte Maximaltemperatur hat. In einer bevorzugten Ausführungsform wird das Umsetzungsprodukt direkt nach Beendigung der Mikrowellenbestrahlung möglichst schnell auf Temperaturen unterhalb 120 °C, bevorzugt unterhalb 100 °C und speziell unterhalb 60 °C abgekühlt. In einer weiteren bevorzugten Ausführungsform wird der Katalysator, sofern anwesend, direkt nach Verlassen des Reaktionsrohres neutralisiert.

Bevorzugt wird die Umsetzung bei Drücken zwischen Atmosphärendruck und 500 bar, besonders bevorzugt zwischen 1,5 bar und 150 bar, insbesondere zwischen 3 bar und 100 bar und speziell zwischen 5 bar und 100 bar wie beispielsweise zwischen 10 bar und 50 bar durchgeführt. Besonders bewährt hat sich das Arbeiten unter erhöhtem Druck, wobei oberhalb der Siedetemperatur (bei Normaldruck) der Edukte, Produkte, des gegebenenfalls anwesenden Lösemittels und/oder oberhalb des während der Reaktion gebildeten Alkohols gearbeitet wird. Besonders bevorzugt wird der Druck so hoch eingestellt, dass das Reaktionsgemisch während der Mikrowellenbestrahlung im flüssigen Zustand verbleibt und nicht siedet. In einer speziellen Ausführungsform wird unter Bedingungen gearbeitet, bei denen der Alkohol (II) überkritisches Verhalten zeigt. So wird beispielsweise die Umesterung mit Methanol in einer bevorzugten Ausführungsform bei Temperaturen oberhalb 240 °C und 81 bar durchgeführt.

Zur Vermeidung von Nebenreaktionen und zur Herstellung von möglichst reinen Produkten hat es sich bewährt, Edukte und Produkte in Gegenwart eines inerten Schutzgases wie beispielsweise Stickstoff, Argon oder Helium zu handhaben.

Obwohl der eingesetzte Alkohol (I) als Lösemittel fungieren kann hat es sich oftmals bewährt, in Gegenwart von weiteren Lösemitteln zu arbeiten um beispielsweise die Viskosität des Reaktionsmediums abzusenken und/oder das Reaktionsgemisch, sofern es heterogen ist, zu fluidisieren. Dafür können prinzipiell alle Lösemittel eingesetzt werden, die unter den angewendeten Reaktionsbedingungen inert sind und nicht mit den Edukten bzw. den gebildeten Produkten reagieren. Ein wichtiger Faktor bei der Auswahl geeigneter weiterer Lösemittel ist deren Polarität, die einerseits die Löseeigenschaften und andererseits das Ausmaß der Wechselwirkung mit Mikrowellenstrahlung bestimmt. Ein besonders wichtiger Faktor bei der Auswahl geeigneter weiterer Lösemittel ist deren dielektrischer Verlust ε". Der dielektrische Verlust ε" beschreibt den Anteil an Mikrowellenstrahlung, der bei der Wechselwirkung einer Substanz mit Mikrowellenstrahlung in Wärme überführt wird. Letzt genannter Wert hat sich als besonders wichtiges Kriterium für die Eignung eines Lösemittels für die Durchführung des erfindungsgemäßen Verfahrens erwiesen.

Besonders bewährt hat sich das Arbeiten in weiteren Lösemitteln, die eine möglichst geringe Mikrowellenabsorption zeigen und somit nur einen kleinen Beitrag zur Erwärmung des Reaktionssystems liefern. Für das erfindungsgemäße Verfahren bevorzugte weitere Lösemittel besitzen einen bei Raumtemperatur und 2450 MHz gemessenen dielektrischen Verlust ε" von weniger als 10 und vorzugsweise weniger als 1 wie beispielsweise weniger als 0,5. Eine Übersicht über den dielektrischen Verlust verschiedener Lösemittel findet sich zum Beispiel in "Microwave Synthesis" von B. L. Hayes, CEM Publishing 2002. Für das erfindungsgemäße Verfahren geeignet sind insbesondere Lösemittel mit ε"-Werten unterhalb 10 wie N-Methylpyrrolidon, N,N-Dimethylformamid oder Aceton, und insbesondere Lösemittel mit ε"-Werten unterhalb 1. Beispiele für besonders bevorzugte Lösemittel mit ε"-Werten unterhalb 1 sind aromatische und/oder aliphatische Kohlenwasserstoffe wie beispielsweise Toluol, Xylol, Ethylbenzol, Tetralin, Hexan, Cyclohexan, Decan, Pentadecan, Dekalin sowie kommerzielle Kohlenwasserstoffgemische wie Benzinfraktionen, Kerosin, Solvent Naphtha, Shellsol^{®} AB, Solvesso^{®} 150, Solvesso^{®} 200, Exxsol^{®}, Isopar^{®} und Shellsol^{®}-Typen. Lösemittelgemische, die ε"-Werte bevorzugt unterhalb 10 und speziell unterhalb 1 aufweisen, sind für die Durchführung des erfindungsgemäßen Verfahrens gleichermaßen bevorzugt.

In einer weiteren bevorzugten Ausführungsform wird das erfindungsgemäße Verfahren in Lösemitteln mit höheren ε"-Werten von beispielsweise 5 und höher wie insbesondere mit ε"-Werten von 10 und höher durchgeführt. Diese Ausführungsform hat sich insbesondere bei der Umsetzung von Reaktionsgemischen bewährt, die selbst, das heißt ohne Anwesenheit von Löse- und/oder Verdünnungsmitteln nur eine sehr geringe Mikrowellenabsorption zeigen. So hat sich diese Ausführungsform insbesondere bei Reaktionsgemischen bewährt, die einen dielektrischen Verlust ε" von weniger als 10 und vorzugsweise weniger als 1 aufweisen. Die durch die Lösemittelzugabe oftmals beobachtete beschleunigte Aufheizung des Reaktionsgemischs erfordert jedoch Maßnahmen zur Einhaltung der Maximaltemperatur.

Sofern in Gegenwart von weiteren Lösemitteln gearbeitet wird, liegt deren Anteil an der Reaktionsmischung bevorzugt zwischen 2 und 95 Gew.-%, speziell zwischen 5 und 90 Gew.-% und insbesondere zwischen 10 und 75 Gew.-% wie beispielsweise zwischen 30 und 60 Gew.-%. Besonders bevorzugt wird das Verfahren ohne Zugabe eines weiteren Lösemittels durchgeführt. Ebenfalls besonders bevorzugt wird das Verfahren mit einem Überschuss des dann auch Lösemittel fungierenden Alkohols (II) durchgeführt.

In einer weiteren bevorzugten Ausführungsform werden dem Reaktionsgemisch in diesem unlösliche, stark Mikrowellen absorbierende Substanzen zugegeben. Diese führen zu einer starken lokalen Erhitzung des Reaktionsgemischs und in der Folge zu weiter beschleunigten Reaktionen. Geeignete derartige Wärmesammler sind beispielsweise Graphit und Borcarbid.

Als Mikrowellen werden elektromagnetische Strahlen mit einer Wellenlänge zwischen etwa 1 cm und 1m und Frequenzen zwischen etwa 300 MHz und 30 GHz bezeichnet. Dieser Frequenzbereich ist prinzipiell für das erfindungsgemäße Verfahren geeignet. Bevorzugt wird für das erfindungsgemäße Verfahren Mikrowellenstrahlung mit den für industrielle, wissenschaftliche, medizinische, häusliche und ähnliche Anwendungen freigegebenen Frequenzen verwendet wie beispielsweise mit Frequenzen von 915 MHz, 2,45 GHz, 5,8 GHz oder 24,12 GHz.

Die für die Durchführung des erfindungsgemäßen Verfahrens in den Hohlraumresonator einzustrahlende Mikrowellenleistung ist insbesondere abhängig von der angestrebten Reaktionstemperatur, aber auch von der Geometrie des Reaktionsrohrs und damit des Reaktionsvolumens sowie der Durchflussgeschwindigkeit des Reaktionsgutes durch das Reaktionsrohr und der Dauer der erforderlichen Bestrahlung. Sie liegt üblicherweise zwischen 200 W und mehreren 100 kW und insbesondere zwischen 500 W und 100 kW wie beispielsweise zwischen 1 kW und 70 kW. Sie kann über einen oder mehrere Mikrowellengeneratoren erzeugt werden.

In einer bevorzugten Ausführungsform wird das Verfahren in einem druckfesten, chemisch inerten Rohr durchgeführt, wobei gegebenenfalls die Edukte sowie Produkte und, sofern anwesend, Lösemittel zu einem Druckaufbau führen können. Nach Beendigung der Reaktion kann der Überdruck durch Entspannung zur Verflüchtigung und Abtrennung von leicht flüchtigen Komponenten sowie gegebenenfalls Lösemittel und/oder zur Abkühlung des Reaktionsprodukts verwendet werden. Das als Nebenprodukt gebildete Polyol (V) wie auch überschüssiger Alkohol (II) können nach dem Abkühlen und/oder Entspannen durch übliche Verfahren wie beispielsweise Phasentrennung, Destillation, Strippen, Flashen und/oder Absorption abgetrennt werden.

Zur Erzielung besonders hoher Umsetzungsgrade hat es sich in vielen Fällen bewährt, das erhaltene Reaktionsprodukt gegebenenfalls nach Austragen von Produkt und/oder Nebenprodukt erneut der Mikrowellenbestrahlung auszusetzen, wobei gegebenenfalls das Verhältnis der eingesetzten Reaktanden um verbrauchte oder unterschüssige Edukte zu ergänzen ist.

Üblicherweise fallen über den erfindungsgemäßen Weg hergestellte Ester in einer für die weitere Verwendung ausreichenden Reinheit an, so dass keine weiteren Auf- oder Nacharbeitungsschritte erforderlich sind. Für spezielle Anforderungen können sie jedoch nach üblichen Reinigungsverfahren wie beispielsweise Destillation, Umkristallisation, Filtration bzw. chromatographische Verfahren weiter aufgereinigt werden.

Die Vorteile des erfindungsgemäßen Verfahrens liegen in einer sehr gleichmäßigen Bestrahlung des Reaktionsgutes im Zentrum eines symmetrischen Mikrowellenfeldes innerhalb eines Reaktionsrohres, dessen Längsachse sich in der Ausbreitungsrichtung der Mikrowellen eines Monomode-Mikrowellenapplikators und insbesondere innerhalb eines E₀₁-Hohlraumresonators beispielsweise mit koaxialem Übergang der Mikrowellen befindet. Dabei erlaubt das erfindungsgemäße Reaktordesign die Durchführung von Reaktionen auch bei sehr hohen Drücken und/oder Temperaturen. Durch Temperatur- und/oder Druckerhöhung wird eine deutliche Steigerung von Umsetzungsgrad und Ausbeute auch gegenüber bekannten Mikrowellenreaktoren beobachtet, ohne dass es dabei zu unerwünschten Nebenreaktionen und/oder Verfärbungen kommt. Überraschenderweise wird dabei ein sehr hoher Wirkungsgrad bei der Ausnutzung der in den Hohlraumresonator eingestrahlten Mikrowellenenergie erzielt, der üblicherweise über 50 %, oftmals über 80 %, teilweise über 90 % und in speziellen Fällen über 95 % wie beispielsweise über 98 % der eingestrahlten Mikrowellenleistung liegt und somit ökonomische wie auch ökologische Vorteile gegenüber konventionellen Herstellverfahren wie auch gegenüber Mikrowellenverfahren des Standes der Technik bietet.

Das erfindungsgemäße Verfahren erlaubt zudem eine kontrollierte, sichere und reproduzierbare Reaktionsführung. Da das Reaktionsgut im Reaktionsrohr parallel zur Ausbreitungsrichtung der Mikrowellen bewegt wird, werden bekannte Überhitzungsphänomene durch unkontrollierbare Feldverteilungen, die zu lokalen Überhitzungen durch wechselnde Intensitäten des Mikrowellenfeldes beispielsweise in Wellenbergen und Knotenpunkten führen können, durch die Fließbewegung des Reaktionsgutes ausgeglichen. Weiterhin können über eine Vergrößerung von Länge und/oder Durchmesser des Reaktionsrohres sowie die Wahl der Wellenlänge der Mikrowellenstrahlung, deren Eindringtiefe in das Reaktionsgut mit abnehmender Frequenz steigt, die auf das Reaktionsgut übertragbare Energie und damit der Durchsatz gesteigert werden. Die genannten Vorteile erlauben es somit, auch mit hohen Mikrowellenleistungen von beispielsweise mehr als 10 kW oder mehr als 100 kW zu arbeiten und somit in Kombination mit einer nur kurzen Verweilzeit im Hohlraumresonator große Produktionsmengen von 100 und mehr Tonnen pro Jahr in einer Anlage zu bewerkstelligen.

Dabei war es überraschend, dass trotz der im kontinuierlich durchflossenen Strömungsrohr nur sehr kurzen Verweildauer des Reaktionsguts im Mikrowellenfeld eine sehr weitgehende Umesterung mit Umsätzen im Allgemeinen von über 80 %, oftmals auch über 90 % und speziell über 95 % wie beispielsweise über 98 % bezogen auf die im Unterschuss eingesetzte Komponente stattfindet, ohne Bildung nennenswerter Mengen an Nebenprodukten. Bei einer entsprechenden Umsetzung dieser Reaktionsgemische in einem Strömungsrohr gleicher Dimensionierung unter thermischer Mantelheizung werden zur Erzielung geeigneter Reaktionstemperaturen extrem hohe Wandtemperaturen benötigt, die zur Bildung gefärbter Spezies führten, aber bei gleichem Zeitintervall nur geringfügige Umesterung bewirken.

Das erfindungsgemäße Verfahren erlaubt somit eine sehr schnelle, energiesparende und kostengünstige Herstellung von Carbonsäureestern in hohen Ausbeuten und mit hoher Reinheit in großtechnischen Mengen. Dabei können auch Polyolester mit einem erhöhten Anteil freier Fettsäuren eingesetzt werden. Bei diesem Verfahren fallen - neben dem Polyol (V) - keine wesentlichen Mengen an Nebenprodukten an. Derartig schnelle und selektive Umsetzungen sind nach klassischen Methoden nicht zu erzielen und waren alleine durch Heizen auf hohe Temperaturen nicht zu erwarten.

### Beispiele

Die Bestrahlungen der Reaktionsgemische mit Mikrowellen erfolgten in einer Apparatur, die als Reaktionsrohr ein Keramikrohr (60 x 1 cm) enthielt, das sich axialsymmetrisch in einem zylindrischen Hohlraumresonator (60 x 10 cm) befand (Bestrahlungszone). An einer der Stirnseiten des Hohlraumresonators verlief dieses Keramikrohr durch den Hohlraum eines als Kopplungsantenne fungierenden Innenleiterrohres. Das von einem Magnetron erzeugte Mikrowellenfeld mit einer Frequenz von 2,45 GHz wurde mittels der Kopplungsantenne in den Hohlraumresonator eingekoppelt (E₀₁-Hohlraumapplikator; Monomode), in dem sich eine stehende Welle ausbildete. Bei Verwendung einer isothermen Reaktionsstrecke wurden die erhitzten Reaktionsgemische unmittelbar nach Verlassen des Reaktionsrohres durch ein thermisch isoliertes Edelstahlrohr (3,0 m x 1 cm, sofern nicht anders angegeben) gefördert. Nach Verlassen des Reaktionsrohres bzw. bei Verwendung der isothermen Reaktionsstrecke nach Verlassen derselbigen wurden die Reaktionsgemische auf Atmosphärendruck entspannt, sofort mittels eines Intensivwärmetauschers auf die angegebenen Temperatur abgekühlt und der Katalysator neutralisiert.

Die Mikrowellenleistung wurde über die Versuchsdauer jeweils in der Art eingestellt, dass die gewünschte Temperatur des Reaktionsgutes am Ende der Reaktionsrohres konstant gehalten wurde. Die in den Versuchsbeschreibungen genannten Mikrowellenleistungen repräsentieren daher den zeitlichen Mittelwert der eingestrahlten Mikroweitenteistung. Die Temperaturmessung des Reaktionsgemischs wurde direkt nach Verlassen des Reaktionsrohres (etwa 15 cm Strecke in einer isolierten Edelstahlkapillare, Ø 1 cm) sowie gegebenenfalls nach Verlassen der Reaktionsstrecke mittels Pt100 Temperatursensor vorgenommen. Vom Reaktionsgemisch nicht direkt absorbierte Mikrowellenenergie wurde an der der Kopplungsantenne entgegen liegenden Stirnseite des Hohlraumresonators reflektiert; die vom Reaktionsgemisch auch beim Rücklauf nicht absorbierte und in Richtung des Magnetrons zurück gespiegelte Mikrowellenenergie wurde mit Hilfe eines Prismensystems (Zirkulator) in ein Wasser enthaltendes Gefäß geleitet. Aus der Differenz zwischen eingestrahlter Energie und Verlustleistung (ermittelt durch die Aufheizung der Wasserlast) wurde die in das Reaktionsgut eingetragene Mikrowellenenergie berechnet.

Mittels einer Hochdruckpumpe und eines geeigneten Druckentlastungsventils wurde die Reaktionsmischung in der Apparatur unter einen solchen Arbeitsdruck gesetzt, der ausreichte, um alle Edukte und Produkte bzw. Kondensationsprodukte stets im flüssigen Zustand zu halten. Die Reaktionsgemische wurden mit einer konstanten Flussrate durch die Vorrichtung gepumpt und die Verweilzeit im Reaktionsrohr (Bestrahlungszone) und Reaktionsstrecke durch Modifizierung der Strömungsgeschwindigkeit eingestellt.

Die Analytik der Produkte erfolgte mittels ¹H-NMR-Spektroskopie bei 500 MHz in CDCl₃.

### Beispiel 1: Herstellung von Cocosfettsäuremethylester

In einem 10 l Büchi-Rührautoklaven mit Gaseinleitungsrohr, Rührer, Innenthermometer und Druckausgleich wurden 3,43 kg Cocosfett (5 mol / Molekulargwicht 686 g/mol) vorgelegt und auf 55 °C erwärmt. Bei dieser Temperatur wurden langsam 1,23 kg Methanol (40 mol) sowie 50 g Natriummethylat als Katalysator zugegeben und unter Rühren homogenisiert.

Das so erhaltene Reaktionsgemisch wurde bei einem Arbeitsdruck von 30 bar kontinuierlich mit 5 I/h durch die Apparatur gepumpt und einer Mikrowellenleistung von 2.0 kW ausgesetzt, von denen 90 % vom Reaktionsgut absorbiert wurden. Die Verweilzeit des Reaktionsgemischs im Reaktionsrohr betrug ca. 34 Sekunden. Am Ende des Reaktionsrohres hatte das Reaktionsgemisch eine Temperatur von 220 °C. Das Reaktionsgemisch wurde direkt nach Verlassen des Reaktionsrohres auf 50 °C abgekühlt und mit Essigsäure neutralisiert.

Das Reaktionsprodukt war leicht gelblich gefärbt. Nach Abtrennung von entstandenem Glycerin und überschüssigem Methanol wurden 3,4 kg Cocosfettsäuremethylester mit einer Reinheit von 98 % erhalten. Der Gehalt an Mono- und Diglyceriden betrug 1,2 bzw. 0,2 Gew.-%.

### Beispiel 2: Herstellung von Rapsfettsäuremethylester

In einem 10 I Büchi-Rührautoklaven mit Gaseinleitungsrohr, Rührer, Innenthermometer und Druckausgleich wurden 4,39 kg Rapsöl (5 mol / Molekulargwicht 878 g/mol) vorgelegt und auf 55 °C erwärmt. Bei dieser Temperatur wurden langsam 1,12 kg Methanol (35 mol) sowie 50 g Natriummethylat als Katalysator zugegeben und unter Rühren homogenisiert.

Das so erhaltene Reaktionsgemisch wurde bei einem Arbeitsdruck von 35 bar kontinuierlich mit 4,5 l/h durch die Apparatur gepumpt und einer Mikrowellenleistung von 1,95 kW ausgesetzt, von denen 92 % vom Reaktionsgut absorbiert wurden. Die Verweilzeit des Reaktionsgemischs in der Bestrahlungszone betrug ca. 38 Sekunden. Beim Verlassen des Reaktionsrohres hatte das Reaktionsgemisch eine Temperatur von 205 °C und wurde direkt mit dieser Temperatur in die isotherme Reaktionsstrecke überführt. Am Ende der Reaktionsstrecke hatte das Reaktionsgemisch eine Temperatur von 195 °C. Das Reaktionsgemisch wurde direkt nach Verlassen der Reaktionsstrecke auf Raumtemperatur abgekühlt und mit Milchsäure neutralisiert.

Das Reaktionsprodukt war leicht gelblich gefärbt. Nach Abtrennung von entstandenem Glycerin und überschüssigem Methanol wurden 4,4 kg Rapsfettsäuremethylester mit einer Reinheit von > 99 % erhalten. Das Produkt enthielt 0,6 Gew.-% Monoglyceride und < 0,2 Gew.-% Diglyceride (Nachweisgrenze).

### Beispiel 3: Herstellung von Rapsfettsäureethylester

In einem 10 I Büchi-Rührautoklaven mit Gaseinleitungsrohr, Rührer, Innenthermometer und Druckausgleich wurden 6,45 kg Rapsöl (7 mol / Molekulargwicht 878 g/mol) vorgelegt und auf 55 °C erwärmt. Bei dieser Temperatur wurden langsam 2,58 kg Ethanol (56 mol) sowie 50 g Natriumethylat als Katalysator zugegeben und unter Rühren homogenisiert.

Das so erhaltene Reaktionsgemisch wurde bei einem Arbeitsdruck von 35 bar kontinuierlich mit 5 I/h durch die Apparatur gepumpt und einer Mikrowellenleistung von 2,2 kW ausgesetzt, von denen 90 % vom Reaktionsgut absorbiert wurden. Die Verweilzeit des Reaktionsgemischs in der Bestrahlungszone betrug ca. 34 Sekunden. Beim Verlassen des Reaktionsrohres hatte das Reaktionsgemisch eine Temperatur von 207 °C und wurde direkt mit dieser Temperatur in die isotherme Reaktionsstrecke überführt. Am Ende der Reaktionsstrecke hatte das Reaktionsgemisch eine Temperatur von 198 °C. Das Reaktionsgemisch wurde direkt nach Verlassen der Reaktionsstrecke auf Raumtemperatur abgekühlt und mit Milchsäure neutralisiert.

Das Reaktionsprodukt war gelblich gefärbt. Nach Abtrennung von entstandenem Glycerin und überschüssigem Ethanol wurden 6,47 kg Rapsfettsäureethylester mit einer Reinheit von 96 % erhalten. Das Produkt enthielt 2,2 Gew.-% Monoglyceride und 0,5 Gew. % Diglyceride.

### Beispiel 4 Herstellung von Rapsfettsäuremethylester aus Rapsöl mit erhöhtem Gehalt an freien Fettsäuren

In einem 10 I Büchi-Rührautoklaven mit Gaseinleitungsrohr, Rührer, Innenthermometer und Druckausgleich wurden 4,62 kg Rapsöl (5 mol / Molekulargewicht 878 g/mol) mit einem Anteil von 5 Gew.-% an freier Rapsölfettsäure (280 g/mol) mit einer Gesamtsäurezahl des Öls von 10 mg KOH/g vorgelegt und auf 55 °C erwärmt. Bei dieser Temperatur wurden langsam 1,12 kg Methanol (35 mol) sowie 50 g Methansulfonsäure als Katalysator zugegeben und unter Rühren homogenisiert.

Das so erhaltene Reaktionsgemisch wurde bei einem Arbeitsdruck von 35 bar kontinuierlich mit 5 I/h durch die Apparatur gepumpt und einer Mikrowellenleistung von 2,1 kW ausgesetzt, von denen 92 % vom Reaktionsgut absorbiert wurden. Die Verweilzeit des Reaktionsgemischs in der Bestrahlungszone betrug ca. 34 Sekunden. Am Ende des Reaktionsrohres hatte das Reaktionsgemisch eine Temperatur von 199 °C. Das Reaktionsgemisch wurde direkt nach Verlassen des Reaktionsrohres auf Raumtemperatur abgekühlt.

Das Reaktionsprodukt war leicht gelblich gefärbt. Nach Abtrennung von entstandenem Glycerin und überschüssigem Methanol wurde durch Waschen mit verdünnter wässriger Natriumhydrogencarbonat-Lösung das Reaktionsprodukt von Katalysatorresten befreit. Die Phasentrennung verlief problemlos, was die Abwesenheit von stark emulgierenden Fettsäuresalzen belegt. Die abgetrennte obere organische Phase hatte eine Restsäurezahl von 0,9 mg KOH/g. Der so hergestellte Rapsfettsäuremethylester enthielt 0,7 Gew.-% Monoglyceride und 0,2 Gew.-% Diglyceride.

## Patentansprüche

1. Kontinuierliches Verfahren zur Herstellung von Estern, in dem mindestens ein Polyolester der Formel (I)
(R¹-COO)ₘR² (I)
worin
R¹ für Wasserstoff oder einen gegebenenfalls substituierten Kohlenwasserstoffrest mit 1 bis 50 Kohlenstoffatomen,
R² für einen gegebenenfalls substituierten Kohlenwasserstoffrest mit 2 bis 10 Kohlenstoffatomen und
m für eine Zahl von 2 bis 10 steht und kleiner oder gleich der Zahl der Kohlenstoffatome in R² ist,
mit mindestens einem einwertigen Alkohol der Formel (II)
R³-OH (II)
worin
R³ für einen gegebenenfalls substituierten Kohlenwasserstoffrest mit 1 bis 30 C-Atomen steht,
unter Mikrowellenbestrahlung in einem Reaktionsrohr, dessen Längsachse sich in der Ausbreitungsrichtung der Mikrowellen eines Monomode-Mikrowellenapplikators befindet, zu mindestens einem Ester der Formel (III)
R¹-COO-R³ (III)
worin
R¹ und R³ die oben angegebenen Bedeutungen haben,
umgesetzt wird.

2. Verfahren nach Anspruch 1, bei dem die Bestrahlung des Reaktionsgemischs mit Mikrowellen in einem weitgehend mikrowellentransparenten Reaktionsrohr innerhalb eines mit einem Mikrowellengenerator über Wellenleiter verbundenen Hohlleiters erfolgt.

3. Verfahren nach einem oder mehreren der Ansprüche 1 und 2, bei dem der Mikrowellenapplikator als Hohlraumresonator ausgestaltet ist.

4. Verfahren nach einem oder mehreren der Ansprüche 1 bis 3, bei dem der Mikrowellenapplikator als Hohlraumresonator vom Reflexionstyp ausgestaltet ist.

5. Verfahren nach einem oder mehreren der Ansprüche 1 bis 4, bei dem das Reaktionsrohr axial mit einer zentralen Symmetrieachse des Hohlleiters fluchtet.

6. Verfahren nach einem oder mehreren der Ansprüche 1 bis 5, bei dem die Bestrahlung des Reaktionsgemisches in einem Hohlraumresonator mit koaxialem Übergang der Mikrowellen erfolgt.

7. Verfahren nach einem oder mehreren der Ansprüche 1 bis 6, bei dem der Hohlraumresonator im E₀₁ₙ-Mode betrieben wird, wobei n eine ganze Zahl von 1 bis 200 ist.

8. Verfahren nach einem oder mehreren der Ansprüche 1 bis 7, bei dem sich im Hohlraumresonator eine stehende Welle ausbildet.

9. Verfahren nach einem oder mehreren der Ansprüche 1 bis 8, bei dem das Reaktionsgut durch die Mikrowellenbestrahlung auf Temperaturen zwischen 80 und 500 °C erhitzt wird.

10. Verfahren nach einem oder mehreren der Ansprüche 1 bis 9, bei dem die Regelung der eingestrahlten Mikrowellenleistung über die Differenz zwischen angestrebter und tatsächlich erreichter Maximaltemperatur des Reaktionsgutes erfolgt.

11. Verfahren nach einem oder mehreren der Ansprüche 1 bis 10, bei dem die Mikrowellenbestrahlung bei Drücken oberhalb des Atmosphärendrucks erfolgt.

12. Verfahren nach einem oder mehreren der Ansprüche 1 bis 11, bei dem das mittels Mikrowellen auf Reaktionstemperatur erhitzte und gegebenenfalls unter Druck stehende Reaktionsgut nach dem Verlassen des Reaktionsrohres direkt in eine sich an das Reaktionsrohr anschließende isotherme Reaktionsstrecke überführt und nach Verlassen der isothermen Reaktionsstrecke abgekühlt wird.

13. Verfahren nach einem oder mehreren der Ansprüche 1 bis 12, bei dem R¹ für einen gegebenenfalls substituierten aliphatischen Kohlenwasserstoffrest mit 2 bis 40 C-Atomen steht.

14. Verfahren nach einem oder mehreren der Ansprüche 1 bis 13, bei dem R¹ für einen Alkyl- oder Alkenylrest steht.

15. Verfahren nach einem oder mehreren der Ansprüche 1 bis 14, bei dem R¹ für einen aliphatischen Kohlenwasserstoffrest mit 7 bis 30 C-Atomen steht.

16. Verfahren nach einem oder mehreren der Ansprüche 1 bis 15, bei dem R² für einen gegebenenfalls substituierten aliphatischen Kohlenwasserstoffrest steht.

17. Verfahren nach einem oder mehreren der Ansprüche 1 bis 16, bei dem der Polyolester (I) ausgewählt ist aus Estern von Ethylenglykol, 1,2-Propandiol, 1,3-Propandiol, Neopentylglykol, Diethylenglykol, Triethylenglykol, Polyethylenglykol, Glycerin, Sorbitan, Sorbitol, Pentaerythrit, Fructose und Glucose.

18. Verfahren nach einem oder mehreren der Ansprüche 1 bis 17, bei dem R³ für einen aliphatischen Rest mit 1 bis 24 C-Atomen steht.

19. Verfahren nach einem oder mehreren der Ansprüche 1 bis 18, bei dem der Alkohol der Formel (II) ausgewählt ist aus Methanol und Ethanol.

20. Verfahren nach einem oder mehreren der Ansprüche 1 bis 18, bei dem R³ für einen mit Heteroatomen unterbrochenen Alkylrest steht.

21. Verfahren nach einem oder mehreren der Ansprüche 1 bis 16, bei dem R³ für eine gegebenenfalls substituierte C₆-C₁₂-Arylgruppe oder eine gegebenenfalls substituierte hetereoaromatische Gruppe mit 5 bis 12 Ringgliedern steht.

22. Verfahren nach einem oder mehreren der Ansprüche 1 bis 21, bei dem 0,001 bis 10 Gew.-%, bezogen auf das Gewicht von Carbonsäureester (I) und Alkohol (II), eines basischen Katalysators eingesetzt wird.

23. Verfahren nach einem oder mehreren der Ansprüche 1 bis 22, bei dem 0,001 bis 10 Gew.-%, bezogen auf das Gewicht von Carbonsäureester (I) und Alkohol (II), eines sauren Katalysators eingesetzt wird.

## Claims

1. A continuous process for preparing esters, in which at least one polyol ester of the formula (I)
(R¹-COO)ₘR² (I)
in which
R¹ is hydrogen or an optionally substituted hydrocarbyl radical having 1 to 50 carbon atoms,
R² is an optionally substituted hydrocarbyl radical having 2 to 10 carbon atoms and
m is a number of from 2 to 10 and is less than or equal to the number of carbon atoms in R²
is reacted with at least one monohydric alcohol of the formula (II)
R³-OH (II)
in which
R³ is an optionally substituted hydrocarbyl radical having 1 to 30 carbon atoms,
under microwave irradiation in a reaction tube whose longitudinal axis is in the direction of propagation of the microwaves from a monomode microwave applicator, to give at least one ester of the formula (III)
R¹-COO-R³ (III)
in which
R¹ and R³ are each as defined above.

2. The process as claimed in claim 1, in which the reaction mixture is irradiated with microwaves in a substantially microwave-transparent reaction tube within a hollow conductor connected via waveguides to a microwave generator.

3. The process as claimed in one or more of claims 1 and 2, in which the microwave applicator is configured as a cavity resonator.

4. The process as claimed in one or more of claims 1 to 3, in which the microwave applicator is configured as a cavity resonator of the reflection type.

5. The process as claimed in one or more of claims 1 to 4, in which the reaction tube is aligned axially with a central axis of symmetry of the hollow conductor.

6. The process as claimed in one or more of claims 1 to 5, in which the reaction mixture is irradiated in a cavity resonator with a coaxial transition of the microwaves.

7. The process as claimed in one or more of claims 1 to 6, in which the cavity resonator is operated in E₀₁ₙ mode where n is an integer from 1 to 200.

8. The process as claimed in one or more of claims 1 to 7, in which a standing wave forms in the cavity resonator.

9. The process as claimed in one or more of claims 1 to 8, in which the reaction mixture is heated by the microwave irradiation to temperatures between 80 and 500°C.

10. The process as claimed in one or more of claims 1 to 9, in which the incident microwave power is regulated via the difference between target and actually attained maximum temperature of the reaction mixture.

11. The process as claimed in one or more of claims 1 to 10, in which the microwave irradiation is effected at pressures above atmospheric pressure.

12. The process as claimed in one or more of claims 1 to 11, in which the optionally pressurized reaction mixture heated to reaction temperature by means of microwaves, after leaving the reaction tube, is transferred directly into an isothermal reaction zone adjoining the reaction tube and, after leaving the isothermal reaction zone, is cooled.

13. The process as claimed in one or more of claims 1 to 12, in which R¹ is an optionally substituted aliphatic hydrocarbyl radical having 2 to 40 carbon atoms.

14. The process as claimed in one or more of claims 1 to 13, in which R¹ is an alkyl or alkenyl radical.

15. The process as claimed in one or more of claims 1 to 14, in which R¹ is an aliphatic hydrocarbyl radical having 7 to 30 carbon atoms.

16. The process as claimed in one or more of claims 1 to 15, in which R² is an optionally substituted aliphatic hydrocarbyl radical.

17. The process as claimed in one or more of claims 1 to 16, in which the polyol ester (I) is selected from esters of ethylene glycol, 1,2-propanediol, 1,3-propanediol, neopentyl glycol, diethylene glycol, triethylene glycol, polyethylene glycol, glycerol, sorbitan, sorbitol, pentaerythritol, fructose and glucose.

18. The process as claimed in one or more of claims 1 to 17, in which R³ is an aliphatic radical having 1 to 24 carbon atoms.

19. The process as claimed in one or more of claims 1 to 18, in which the alcohol of the formula (II) is selected from methanol and ethanol.

20. The process as claimed in one or more of claims 1 to 18, in which R³ is an alkyl radical interrupted by heteroatoms.

21. The process as claimed in one or more of claims 1 to 16, in which R³ is an optionally substituted C₆-C₁₂-aryl group or an optionally substituted heteroaromatic group having 5 to 12 ring members.

22. The process as claimed in one or more of claims 1 to 21, in which 0.001 to 10% by weight, based on the weight of carboxylic ester (I) and alcohol (II), of a basic catalyst is used.

23. The process as claimed in one or more of claims 1 to 22, in which 0.001 to 10% by weight, based on the weight of carboxylic ester (I) and alcohol (II), of an acidic catalyst is used.

## Revendications

1. Procédé continu pour la préparation d'esters, dans lequel on transforme au moins un polyolester de formule (I)
(R¹-COO)ₘR² (I)
où
R¹ représente hydrogène ou un radical hydrocarboné le cas échéant substitué comprenant 1 à 50 atomes de carbone,
R² représente un radical hydrocarboné le cas échéant substitué comprenant 2 à 10 atomes de carbone et
m vaut un nombre de 2 à 10 et est inférieur ou égal au nombre d'atomes de carbone dans R²,
avec au moins un alcool monovalent de formule (II)
R³-OH (II)
où
R³ représente un radical hydrocarboné le cas échéant substitué comprenant 1 à 30 atomes de carbone,
sous une irradiation par des micro-ondes dans un tube de réaction, dont l'axe longitudinal se trouve dans le sens de la propagation des micro-ondes d'un dispositif d'application de micro-ondes monomodales, en au moins un ester de formule (III)
R¹-COO-R³ (III)
où
R¹ et R³ présentent les significations indiquées ci-dessus.

2. Procédé selon la revendication 1, dans lequel l'irradiation du mélange réactionnel par des micro-ondes a lieu dans un tube de réaction dans une large mesure transparent aux micro-ondes, dans un conducteur creux relié via des guides d'ondes à un générateur de micro-ondes.

3. Procédé selon l'une ou plusieurs des revendications 1 et 2, dans lequel le dispositif d'application de micro-ondes est réalisé sous forme d'une cavité résonante.

4. Procédé selon l'une ou plusieurs des revendications 1 à 3, dans lequel le dispositif d'application de micro-ondes est réalisé sous forme d'une cavité résonante de type réflexion.

5. Procédé selon l'une ou plusieurs des revendications 1 à 4, dans lequel le tube de réaction est axialement aligné sur un axe de symétrie central du conducteur creux.

6. Procédé selon l'une ou plusieurs des revendications 1 à 5, dans lequel l'irradiation du mélange réactionnel a lieu dans une cavité résonante avec une transition coaxiale des micro-ondes.

7. Procédé selon l'une ou plusieurs des revendications 1 à 6, dans lequel la cavité résonante est exploitée dans un mode E₀₁ₙ, n représentant un nombre entier de 1 à 200.

8. Procédé selon l'une ou plusieurs des revendications 1 à 7, dans lequel une onde stationnaire se forme dans la cavité résonante.

9. Procédé selon l'une ou plusieurs des revendications 1 à 8, dans lequel le produit de réaction est chauffé à des températures entre 80 et 500 °C par l'irradiation par des micro-ondes.

10. Procédé selon l'une ou plusieurs des revendications 1 à 9, dans lequel la régulation de la puissance irradiée des micro-ondes a lieu via la différence entre la température maximale visée et la température maximale effectivement atteinte du produit de réaction.

11. Procédé selon l'une ou plusieurs des revendications 1 à 10, dans lequel l'irradiation par des micro-ondes a lieu à des pressions supérieures à la pression atmosphérique.

12. Procédé selon l'une ou plusieurs des revendications 1 à 11, dans lequel le produit de réaction chauffé à la température de réaction au moyen de micro-ondes et se trouvant le cas échéant sous pression est transféré, après avoir quitté le tube de réaction, directement dans une zone de réaction isothermique consécutive au tube de réaction et est refroidi après avoir quitté la zone de réaction isothermique.

13. Procédé selon l'une ou plusieurs des revendications 1 à 12, dans lequel R¹ représente un radical hydrocarboné aliphatique le cas échéant substitué comprenant 2 à 40 atomes de carbone.

14. Procédé selon l'une ou plusieurs des revendications 1 à 13, dans lequel R¹ représente un radical alkyle ou alcényle.

15. Procédé selon l'une ou plusieurs des revendications 1 à 14, dans lequel R¹ représente un radical hydrocarboné aliphatique comprenant 7 à 30 atomes de carbone.

16. Procédé selon l'une ou plusieurs des revendications 1 à 15, dans lequel R² représente un radical hydrocarboné aliphatique le cas échéant substitué.

17. Procédé selon l'une ou plusieurs des revendications 1 à 16, dans lequel le polyolester (I) est choisi parmi les esters d'éthylèneglycol, de 1,2-propanediol, de 1,3-propanediol, de néopentylglycol, de diéthylèneglycol, de triéthylèneglycol, de polyéthylèneglycol, de glycérol, de sorbitane, de sorbitol, de pentaérythritol, de fructose et de glucose.

18. Procédé selon l'une ou plusieurs des revendications 1 à 17, dans lequel R³ représente un radical aliphatique comprenant 1 à 24 atomes de carbone.

19. Procédé selon l'une ou plusieurs des revendications 1 à 18, dans lequel l'alcool de formule (II) est choisi parmi le méthanol et l'éthanol.

20. Procédé selon l'une ou plusieurs des revendications 1 à 18, dans lequel R³ représente un radical alkyle interrompu par des hétéroatomes.

21. Procédé selon l'une ou plusieurs des revendications 1 à 16, dans lequel R³ représente un groupe C₆-C₁₂-aryle le cas échéant substitué ou un groupe hétéroaromatique le cas échéant substitué comprenant 5 à 12 chaînons.

22. Procédé selon l'une ou plusieurs des revendications 1 à 21, dans lequel on utilise 0,001 à 10% en poids, par rapport au poids de l'ester de l'acide carboxylique (I) et de l'alcool (II), d'un catalyseur basique.

23. Procédé selon l'une ou plusieurs des revendications 1 à 22, dans lequel on utilise 0,001 à 10% en poids, par rapport au poids de l'ester de l'acide carboxylique (I) et de l'alcool (II), d'un catalyseur acide.
